# EUROPEAN PATENT APPLICATION

(11) **EP 1 941 861 A1**
(43) Date of publication of application: **09.07.2008**
(21) Application number: 06798055.7
(22) Date of filing: 15.09.2006
(51) Int. Cl.: A61K 8/44, A61K 8/42, A61K 8/46, A61K 8/49, A61Q 19/08

(54) **WRINKLE-IMPROVING AGENT**

(30) Priority: 22.09.2005 JP 2005275535
(71) Applicant: Shiseido Company, Limited, Chuo-ku Tokyo 104-8010 (JP)
(72) Inventor: KAMINUMA, Mikiko, Yokohama-shi, Kanagawa, 224-8558 (JP); SUETSUGU, Masaru, Yokohama-shi, Kanagawa, 224-8558 (JP); TSUNENAGA, Makoto, Yokohama-shi, Kanagawa, 224-8558 (JP); HASEGAWA, Kiyotaka, Yokohama-shi, Kanagawa, 224-8558 (JP); INOMATA, Shinji, Yokohama-shi, Kanagawa, 224-8558 (JP)
(74) Representative: Merkle, Gebhard
(86) International application number: PCT/JP2006/318392
(87) International publication number: WO 2007/034750

(57) **Abstract**

The invention provides a novel wrinkle-improving agent that has an effect of improving wrinkles, that does not entail problems of safety or pain even when applied to the skin, and that contains a very safe substance as an active component. A discovery was made that N-benzoyl-β-alanine, other specific β-alanine derivatives, and salts thereof have a wrinkle-improving effect, whereby a wrinkle-improving agent containing as an active component one, two, or more types of compounds selected from the group composed of these specific β-alanine derivatives and the salts thereof was developed.

## Description

### TECHNICAL FIELD

The present invention relates to a wrinkle-improving agent that has an effect of improving wrinkles, and particularly relates to the active component of the wrinkle-improving agent.

### BACKGROUND ART

Aging progresses in all the organs of the body, but among these organs, the skin is visible to the eye. The face in particular readily draws attention, and the occurrence of wrinkles and fine lines on the face and skin in connection with aging is troublesome to many middle-aged and elderly people, especially women. The need for wrinkle-improving cosmetics has conventionally been keenly felt, but numerous aspects of the mechanisms involved in aging and wrinkling have remained unclear, and therefore conventional cosmetics have not progressed beyond selecting from methods for mixing synthetic polymer products and mucopolysaccharides, collagen, or other biochemical products and attempting to maintain water content in order to improve wrinkles.

However, wrinkling and aging of the skin cannot be adequately prevented using the aforementioned methods alone. Research on aging has advanced in recent years. Macroscopically speaking, advancing age is an important factor in the aging of the skin. Dryness, oxidation, sunlight (ultraviolet rays), and the like are also examples of factors directly relates to skin aging. Among these factors, sunlight (ultraviolet rays) is clearly important in those changes referred to as "photoaging" The face is the site of the body most prone to the advance of photoaging, but it has become clear that collagen fibers, which are the primary matrix components of the dermis, are markedly reduced in photoaged skin. A close relationship has been suggested between decreases in collagen fiber and phenomena such as loss of firmness and the occurrence of wrinkles and fine lines. Thus, in wrinkling and aging skin, the growth activity of fibroblasts, which are important cells in the dermis, and the function for synthesizing collagen and the like are reduced, and the turnover rate of collagen and the like also slows in connection with a variety of skin-aging factors, particularly exposure to sunlight (ultraviolet rays). As a result, the elasticity of the skin is lost, wrinkles occur, and aging of the skin progresses.

Alanine, which is an amino acid, is known as a substance that is effective in improving wrinkles (see Patent Documents 1 and 2). However, the use of alanine is problematic in that the application of alanine to the skin is extremely painful. Among the β-alanine derivatives and salts thereof used in the present invention, N-benzoyl-β-alanine is a known substance and is known to be effective in reducing the nephrotoxicity of antibiotics (see Patent Document 3), but this compound has not been used as a wrinkle-improving agent.

Patent Document 1: Japanese Laid-Open Patent Application No. 11-49628
Patent Document 2: Japanese Laid-Open Patent Application No. 8-99862
Patent Document 3: Japanese Laid-Open Patent Application No. 62-174015

### DISCLOSURE OF THE INVENTION

### PROBLEMS TO BE SOLVED BY THE INVENTION

The present invention was derived in light of the above-mentioned circumstances, and an object thereof is to provide a novel wrinkle-improving agent that has an effect of improving wrinkles, that does not have problems of safety and not accompanied by pain when it is applied to the skin, and that contains a very safe substance as an active component.

### MEANS USED TO SOLVE THE ABOVE-MENTIONED PROBLEMS

As a result of thorough-going research in order to solve the above-mentioned problems, the inventors arrived at the completion of the present invention after discovering that specific β-alanine derivatives and salts thereof have an effect of improving wrinkles, are very safe, and solve the above-mentioned problems.

Specifically, the present invention provides a wrinkle-improving agent comprising as an active component one, two, or more compounds selected from β-alanine derivatives represented by the following general formula (1), (2) or (3), and salts thereof.

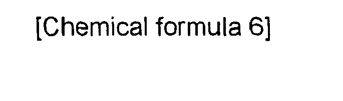

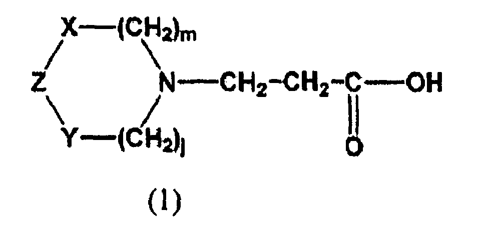

(In general formula (1), X and Y each independently represent an oxygen atom (O), a nitrogen atom (N), a CHQ group (in the group, Q represents a hydrogen atom (H) or a C₁₋₃ alkyl group or hydroxyl group), or a carbonyl group (C=O). Z represents a methylene group (CH₂), an ethylene group (CH₂-CH₂), CH=CH, or a benzene ring. j and m each independently represent an integer of 0 to 5. The total of j and m (j + m) is 0 to 6.)

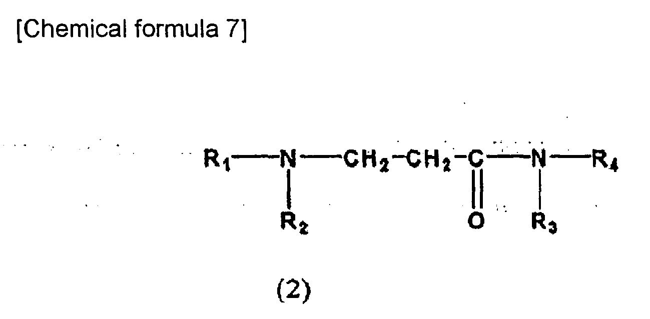

(In general formula (2), R₁ and R₂ each independently represent a hydrogen atom, C₁₋₃ alkyl group, or C₁₋₃ alkenyl group. R₃ and R₄ each independently represent a hydrogen atom, a C₁₋₃ alkyl group, a C₁₋₃ alkenyl group, a cyclohexyl group, or a group containing nitrogen atoms and forming a cyclic structure from R₃ and R₄. The total number of carbons in R₃ and R₄ is 0 to 6.)

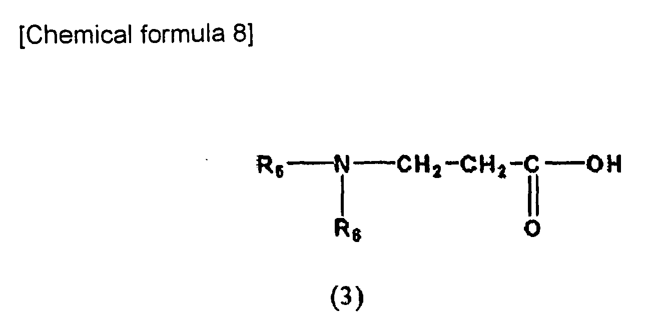

[In general formula (3), R₅ and R₆ are each independently a hydrogen atom, C₁₋₇ alkyl group, C₁₋₇ alkenyl group, C₃₋₇ hydrocarbon group having a cyclic segment, pyridyl group, cyclohexyl carbonyl group, cyclopentyl carbonyl group, nicotinoyl group, isonicotinoyl group, picolinoyl group, nipecotinoyl group, isonipecotinoyl group, *N*-acetyl nipecotinoyl group, *N*-acetyl isonipecotinoyl group, benzyloxycarbonyl group, group represented by general formula (4)

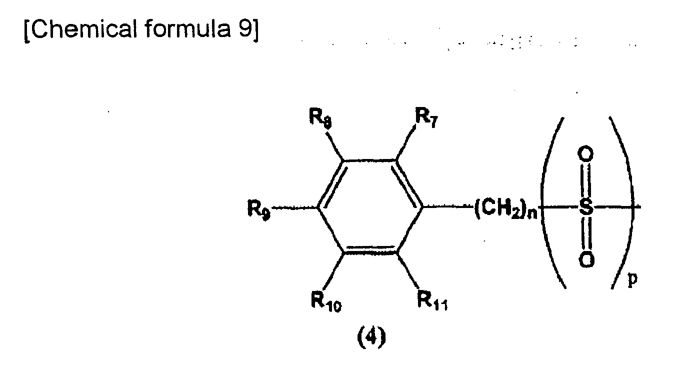

(In general formula (4), R₇ through R₁₁ each independently represent a hydrogen atom, hydroxyl group, C₁₋₄ alkyloxy group, C₁₋₄ alkenyloxy group, C₁₋₃ alkyl group, or C₁₋₃ alkenyl group; n represents an integer of 0 to 2; and p represents an integer .of. 0 or 1.), or a group represented by general formula (5)

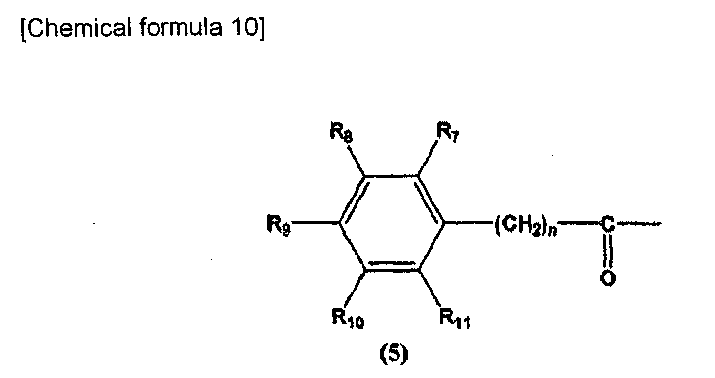

(in general formula (5), R₇ through R₁₁ and n are the same as in general formula (4)). R₅ and R₆ cannot both be hydrogen atoms.]

The β-alanine derivative is preferably N-benzoyl-β-alanine.

"Improvement" of wrinkles in the present invention is a concept that also includes functions for minimizing the occurrence of wrinkles.

### EFFECT OF THE INVENTION

The β-alanine derivative that is the active component of the present invention is very safe, has a strong wrinkle-improving effect, is useful as a wrinkle-improving agent, and, by applying the wrinkle-improving agent to the skin, can bring about wrinkle improvement.

### BRIEF DESCRIPTION OF THE DRAWINGS

FIG. 1 is a diagram that shows the degree of wrinkle improvement from the application of *N*-benzoyl-β-alanine, which is an example of the effective component of the present invention; and
FIG. 2 displays the changes in wrinkle area ratio and wrinkle volume ratio from the application of benzoyl-β-alanine.

### BEST MODE FOR CARRYING OUT THE INVENTION

The present invention will now be described in detail.

The present invention uses as an active component one, two, or more compounds selected from β-alanine derivatives represented by the following general formula (1), (2) or (3), and salts thereof.

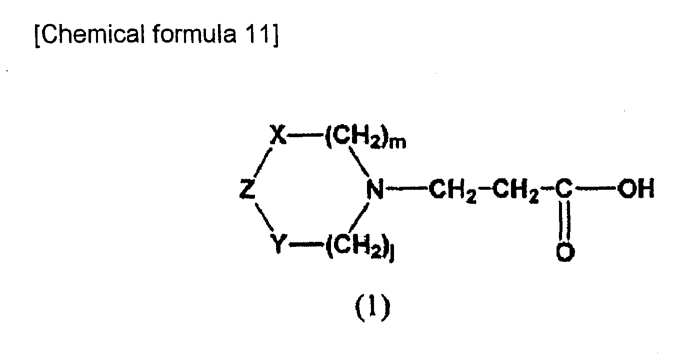

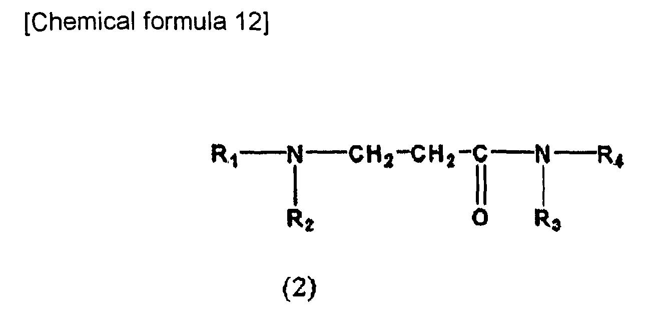

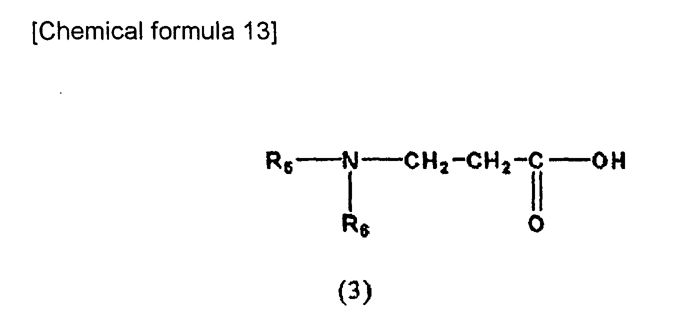

The β-alanine derivative represented by general formula (1) according to the present invention will now be described.

X and Y in general formula (1) each independently represent an oxygen atom (O), a nitrogen atom (N), a CHQ group (in the formula, Q is a hydrogen atom (H), C₁₋₃ alkyl group, or hydroxyl group), or a carbonyl group (C=O). Specific examples of the CHQ group are CH₂, CH(CH₃), CH(CH₂CH₃), CH(CH₂CH₂CH₃), CH(CH(CH₃)₂), and CH(OH) groups. It is particularly preferred that X and Y each independently be a methylene group (CH₂) or oxygen atom (O) because the compound will be easily synthesized. Z is a methylene group (CH₂), an ethylene group (CH₂-CH₂), CH=CH, or a benzene ring. In cases where Z is a benzene ring, the substitution positions of X and Y on the benzene ring are not particularly restricted. j and m each independently represent an integer of 0 to 5. The total of j and m (j + m) is 0 to 6.

Specific examples of the β-alanine derivative represented by general formula (1) are 3-(2'-piperidone)-propionic acid, 3-(4'-piperidone)-propionic acid, 3-(2'-pyrrolidone)-propionic acid, 3-(2'-hydroxypiperidine)-propionic acid, 3-(3'-hydroxypiperidine)-propionic acid, 3-(4'-hydroxypiperidino)-propionic acid, 3-(2'-oxo-1'-hexamethyleneiminyl)-propionic acid, 3-(1'-pyrrolidine)-propionic acid, 3-(1'-piperidine)-propionic acid, 3-(1'-hexamethyleneiminyl)-propionic acid, 3-morpholinopropionic acid, 3-(piperazin-1'-yl)propionic acid, 3-(2',5'-dioxo-1'-pyrrolidin-1'-yl)propionic acid, 3-(2',6'-dioxo-1'-piperidin-1'-yl)propionic acid, 3-(1'-aza-2',5'-dioxo-3'-cyclopenten-1'-yl)propionic acid, and *N*-(2'-carboxyethyl)phthalimide.

Examples of the β-alanine derivative represented by general formula (2) according to the present invention will now be described.

R₁ and R₂ in general formula (2) are each independently a hydrogen atom, C₁₋₃ alkyl group, or C₁₋₃ alkenyl group. Specific examples of C₁₋₃ alkyl and alkenyl groups are methyl, ethyl, *n*-propyl, *iso*-propyl, and allyl groups. It is preferred that one or both of R₁ and R₂ be a hydrogen atom because the effect of the present invention will be good. It is further preferred that they both be hydrogen atoms.

R₃ and R₄ each independently represent a hydrogen atom, a C₁₋₃ alkyl group, a C₁₋₃ alkenyl group, a cyclohexyl group, or a group containing nitrogen atoms and forming a cyclic structure from R₃ and R₄. The total number of carbon atoms in R₃ and R₄ is 0 to 6. Specific examples of C₁₋₃ alkyl or alkenyl groups are methyl, ethyl, *n*-propyl, *iso*-propyl, and allyl groups. Specific examples of the group containing nitrogen atoms and forming a cyclic structure from R₃ and R₄ are pyrrolidinyl, piperidinyl, and hexamethyleneiminyl groups. It is preferred that one or both of R₃ and R₄ be a hydrogen atom because the effects of the present invention will be good. It is further preferred that they both be a hydrogen atom. It is most preferable that each of R₁, R₂, R₃, and R₄ in general formula (2) be a hydrogen atom.

Specific examples of the β-alanine derivative represented by general formula (2) are β**-**alanine amide, β-alanine methylamine amide, β-alanine dimethylamine amide, β-alanine ethylamine amide, β-alanine diethylamine amide, β-alanine(*N*'-methyl-*N*'-ethylamine)amide, β-alanine *n*-propylamine amide, β-alanine di *n-*propylamine amide, β-alanine(*N*'-methyl-*N*'-*n*-propylamine)amide, β-alanine(*N*'-ethyl-*N*'-*n*-propylamine)amide, β-alanine *iso-*propylamine amide, β-alanin di-*iso-*propylamine amide, β-alanine (*N*'-methyl-*N*'-*iso*-propylamine)amide, β-alanine (*N*'-ethyl-*N*'-*iso*-propylamine)amide, β-alanine(*N*'-*n*-propyl*-N*'-*iso*-propylamine)amide, β-alanine allylamine amide, β**-**alanine diallylamine amide, β-alanine(*N*'-methyl-*N*'-allylamine)amide, β-alanine(*N*'-ethyl-*N*'-allylamine)amide, β-alanine(*N*'-*n*-propyl-*N*'-allylamine)amide, β-alanine(*N*'-iso-propyl-*N*'-allylamine)amide, β-alanine cyclohexylamine amide, β-alanine piperidine amide, β-alanine pyrrolidine amide, β-alanine hexamethylene imine amide, *N*-methyl-3-aminopropionic acid amide, *N-*methyl-3-aminopropionic acid methylamine amide, *N*-methyl-3-aminopropionic acid dimethylamine amide, *N*-methyl-3-aminopropionic acid ethylamine amide, *N*-methyl-3-aminopropionic acid diethyl amine amide, *N*-methyl-3-aminopropionic acid (*N*'-methyl-*N*'-ethylamine)amide, *N*-methyl-3-aminopropionic acid *n*-propylamine amide,

*N*-methyl-3-aminopropionic acid di-*n*-propylamine amide, *N*-methyl-3-aminopropionic acid (*N*'-methyl-*N*'-*n*-propylamine)amide, *N*-methyl-3-aminopropionic acid (*N*'-ethyl-*N'*-*n*-propylamine)amide, *N*-methyl-3-aminopropionic acid *iso-*propylamine amide, *N*-methyl-3-aminopropionic acid di-*iso*-propylamine amide, N-methyl-3-aminopropionic acid (*N*'-methyl-*N*'-*iso*-propylamine)amide, *N*-methyl-3-aminopropionic acid (*N*'-ethyl-*N*'-*iso*-propylamine)amide, *N*-methyl-3-aminopropionic acid (*N*'-n-propyl-N'-*iso-*propylamine)amide, *N*-methyl-3-aminopropionic acid allylamine amide, *N*-methyl-3-aminopropionic acid diallylamine amide, *N*-methyl-3-aminopropionic acid (*N*'-methyl-*N*'-allylamine)amide, *N*-methyl-3-aminopropionic acid (*N*'-ethyl-*N*'-allylamine)amide, *N*-methyl-3-aminopropionic acid (*N*'-n-propyl-*N*'-allylamine)amide, *N*-methyl-3-aminopropionic acid (*N*'-*iso*-propyl-*N*'-allylamine)amide, *N*-methyl-3-aminopropionic acid cyclohexylamine amide, *N*-methyl-3-aminopropionic acid piperidine amide, *N*-methyl-3-aminopropionic acid pyrrolidine amide, *N*-methyl-3-aminopropionic acid hexamethylene imine amide, *N*-ethyl-3-aminopropionic acid amide, *N*-ethyl-3-aminopropionic acid methyl amide, *N*-ethyl-3-aminopropionic acid dimethyl amine amide, *N*-ethyl-3-aminopropionic acid ethyl amine amide, *N*-ethyl-3-aminopropionic acid diethyl amine amide, *N*-ethyl-3-aminopropionic acid (*N*'-methyl-*N*'-ethylamine)amide,

*N*-ethyl-3-aminopropionic acid n-propylamine amide, *N*-ethyl-3-aminopropionic acid di-n-propylamine amide, *N*-ethyl-3-aminopropionic acid (*N*'-methyl-*N*'-*n*-propylamine)amide, *N*-ethyl-3-aminopropionic acid (*N*'-ethyl-*N*'-*n-*propylamine)amide, *N*-ethyl-3-aminopropionic acid *iso*-propylamine amide, *N*-ethyl-3-aminopropionic acid di-*iso*-propylamine amide, *N*-ethyl-3-aminopropionic acid (*N*'-methyl-*N*'-*iso*-propylamine)amide, N-ethyl-3-aminopropionic acid (*N*'-ethyl-*N*'-*iso-*propylamine)amide, *N*-ethyl-3-aminopropionic acid (*N*'-*n*-propyl-*N*'-*iso-*propylamine)amide, *N*-ethyl-3-aminopropionic acid allylamine amide, *N*-ethyl-3-aminopropionic acid diallylamine amide, *N*-ethyl-3-aminopropionic acid (*N*'-methyl-*N*'-allylamine)amide, *N*-ethyl-3-aminopropionic acid (*N*'-ethyl-*N*'-allylamine)amide, *N-*ethyl-3-aminopropionic acid (*N*'-*n*-propyl-*N*'-allylamine)amide, *N*-ethyl-3-aminopropionic acid (*N*'-*iso*-propyl-*N*'-allylamine)amide, *N*-ethyl-3-aminopropionic acid cyclohexylamine amide, *N*-ethyl-3-aminopropionic acid piperidine amide, *N-*ethyl-3-aminopropionic acid pyrrolidine amide, *N*-ethyl-3-aminopropionic acid hexamethylene imine amide, *N*-*n*-propyl-3-aminopropionic acid amide, *N*-*n*-propyl-3-aminopropionic acid methyl amide, *N-n*-propyl-3-aminopropionic acid dimethylamine amide, *N-n-*propyl-3-aminopropionic acid ethylamine amide, *N-n*-propyl-3-aminopropionic acid diethylamine amide, *N-n*-propyl-3-aminopropionic acid (*N*'-Methyl-*N*'-ethylamine)amide.

*N-n*-propyl-3-aminopropionic acid *n*-propylamine amide, *N*-*n*-propyl-3-aminopropionic acid di-*n*-propylamine amide, *N-n*-propyl-3-aminopropionic acid (*N*'-methyl-*N*'-*n*-propylamine)amide, *N-n*-propyl-3-aminopropionic acid (*N*'-ethyl-*N*'-*n-*propylamine)amide, *N-n*-propyl-3-aminopropionic acid *iso*-propylamine amide, *N*-*n-*propyl-3-aminopropionic acid di-*iso*-propylamine amide, *N-n*-propyl-3-aminopropionic acid (*N*'-methyl-*N*'-*iso*-propylamine)amide, *N-n*-propyl-3-aminopropionic acid (*N*'-ethyl-*N*'-*iso*-propylamine)amide, *N-n*-propyl-3-aminopropionic acid (*N*'-*n*-propyl-*N*'-*iso-*propylamine)amide, *N-n*-propyl-3-aminopropionic acid allylamine amide, *N-n*-propyl-3-aminopropionic acid diallylamine amide, *N-n-*propyl-3-aminopropionic acid (*N*'-methyl-*N*'-allylamine)amide, *N-n*-propyl-3-aminopropionic acid (*N*'-ethyl-*N*'-allylamine)amide, *N*-*n*-propyl-3-aminopropionic acid (*N*'-*n*-propyl-*N*'-allylamine)amide, *N-n*-propyl-3-aminopropionic acid (*N*'-*iso*-propyl-*N*'-allylamine)amide, *N-n*-propyl-3-aminopropionic acid cyclohexylamine amide, *N-n*-propyl-3-aminopropionic acid piperidine amide, *N-n-*propyl-3-aminopropionic acid pyrrolidine amide, *N*-*n*-propyl-3-aminopropionic acid hexamethyleneimine amide, *N-iso*-propyl-3-aminopropionic acid amide, *N-iso*-propyl-3-aminopropionic acid methyl amide,

*N-iso*-propyl-3-aminopropionic acid dimethylamine amide, *N*-*iso*-propyl-3-aminopropionic acid ethylamine amide, *N-iso*-propyl-3-aminopropionic acid diethylamine amide, *N-iso-*propyl-3-aminopropionic acid (*N*'-methyl-*N*'-ethylamine)amide, *N-iso*-propyl-3-aminopropionic acid *n*-propylamine amide, *N*-*iso-*propyl-3-aminopropionic acid di-*n*-propylamine amide, *N-iso*-propyl-3-aminopropropionic acid (*N*'-methyl-*N*'-*n*-propylamine)amide, *N-iso*-propyl-3-aminopropionic acid (*N*'-ethyl-*N*'-*n-*propylamine)amide, *N-iso-*propyl-3-aminopropionic acid *iso-*propylamine amide, *N-iso*-propyl-3-aminopropionic acid di-*iso-*propylamine amide, *N*-*iso*-propyl-3-aminopropionic acid (*N*'-methyl-*N*'-*iso*-propylamine)amide, *N*-*iso*-propyl-3-aminopropionic acid (*N*'-ethyl-*N*'-*iso-*propylamine)amide, *N*-*iso*-propyl-3-aminopropionic acid (*N*'-*n*-propyl-*N*'-*iso*-propylamine)amide, *N*-*iso*-propyl-3-aminopropionic acid allylamine amide, *N*-*iso*-propyl-3-aminopropionic acid diallylamine amide, *N-iso*-propyl-3-aminopropionic acid (*N*'-methyl-*N*'-allylamine)amide, *N*-*iso*-propyl-3-aminopropionic acid (*N*'-ethyl-*N*'-allylamine)amide, *N-iso*-propyl-3-aminopropionic acid (*N*'-*n*-propyl-*N*'-allylamine)amide, *N-iso*-propyl-3-aminopropionic acid (*N*'-*iso*-propyl-*N*'-allylamine)amide, *N-iso*-propyl-3-aminopropionic acid cyclohexylamine amide,

*N-iso*-propyl-3-aminopropionic acid piperidine amide, *N*-*iso*-propyl-3-aminopropionic acid pyrrolidine amide, *N-iso*-propyl-3-aminopropionic acid hexamethyleneimine amide, *N-*allyl-3-aminopropionic acid amide, *N*-allyl-3-aminopropionic acid methyl amide, *N*-allyl-3-aminopropionic acid dimethylamine amide, *N*-allyl-3-aminopropionic acid ethylamine amide, *N*-allyl-3-aminopropionic acid diethylamine amide, *N*-allyl-3-aminopropionic acid (*N*'-methyl-*N*'-ethylamine)amide, *N*-allyl-3-aminopropionic acid *n*-propylamine amide, *N*-allyl-3-aminopropionic acid di-*n*-propylamine amide, *N*-allyl-3-aminopropionic acid (*N*'-methyl-*N*'-*n*-propylamine)amide, *N*-allyl-3-aminopropionic acid (*N*'-ethyl-*N*'-*n-*propylamine)amide, *N*-allyl-3-aminopropionic acid *iso-*propylamine amide, *N*-allyl-3-aminopropionic acid di-*iso*-propylamine amide, *N*-allyl-3-aminopropionic acid (*N*'-methyl-*N*'-*iso-*propylamine)amide, *N*-allyl-3-aminopropionic acid (*N*'-ethyl-*N*'-*iso-*propylamine)amide, *N*-allyl-3-aminopropionic acid (*N*'-*n*-propyl-*N*'-*iso-*propylamine)amide, *N*-allyl-3-aminopropionic acid allylamine amide, *N*-allyl-3-aminopropionic acid diallylamine amide, *N*-allyl-3-aminopropionic acid (*N*'-methyl-*N*'-allylamine)amide, *N*-allyl-3-aminopropionic acid (*N*'-ethyl-*N*'-allylamine)amide, *N-*allyl-3-aminopropionic acid (*N*'-*n*-propyl-*N*'-allylamine)amide,

*N*-allyl-3-aminopropionic acid (*N*'-*iso*-propyl-*N*'-allylamine)amide, *N*-allyl-3-aminopropionic acid cyclohexylamine amide, *N*-allyl-3-aminopropionic acid piperidine amide, *N*-allyl-3-aminopropionic acid pyrrolidine amide, *N*-allyl-3-aminopropionic acid hexamethyleneimine amide, *N,N*-dimethyl-3-aminopropionic acid amide, *N,N-*dimethyl-3-aminopropionic acid methyl amide, *N,N-*dimethyl-3-aminopropionic a0cid dimethylamine amide, *N*,*N*-dimethyl-3-aminopropionic acid ethylamine amide, *N*,*N-*dimethyl-3-aminopropionic acid diethylamine amide, *N*,*N*-dimethyl-3-aminopropionic acid (*N*'-methyl-*N*'-ethylamine)amide, *N*,*N*-dimethyl-3-aminopropionic acid *n-*propylamine amide, *N,N*-dimethyl-3-aminopropionic acid di-*n*-propylamine amide, *N*,*N*-dimethyl-3-aminopropionic acid (*N*'-methyl-*N*'-*n*-propylamine)amide, *N*,*N-*dimethyl-3-aminopropionic acid (*N*'-ethyl-*N*'-*n*-propylamine)amide, *N,N*-dimethyl-3-aminopropionic acid *iso*-propylamine amide, *N*,*N-*dimethyl-3-aminopropionic acid di-*iso*-propylamine amide, *N*,*N*-dimethyl-3-aminopropionic acid (*N*'-methyl-*N*'-*iso-*propylamine)amide, *N*,*N*-dimethyl-3-aminopropionic acid (*N*-ethyl-*N*-iso-propylamine)amide, *N,N*-dimethyl-3-aminopropionic acid (*N*-*n*-propyl-*N*'-*iso-*propylamine)amide, *N*,*N*-dimethyl-3-aminopropionic acid allylamine amide, *N*,*N-*dimethyl-3-aminopropionic acid diallylamine amide, *N*,*N*-dimethyl-3-aminopropionic acid (*N*'-methyl-*N*'-allylamine)amide,

*N*,*N*-dimethyl-3-aminopropionic acid (*N*'-ethyl-*N*'-allylamine)amide, *N,N-*dimethyl-3-aminopropionic acid (*N*'-*n*-propyl-*N*'-allylamine)amide, *N*,*N*-dimethyl-3-aminopropionic acid (*N*'-*iso*-propyl-*N*'-allylamine)amide, *N*,*N*-dimethyl-3-aminopropionic acid cyclohexylamine amide, *N*,*N*-dimethyl-3-aminopropionic acid piperidine amide, *N*,*N*-dimethyl-3-aminopropionic acid pyrrolidine amide, *N,N-*dimethyl-3-aminopropionic acid hexamethyleneimine amide, *N*,*N*-diethyl-3-aminopropionic acid amide, *N*,*N*-diethyl-3-aminopropionic acid methyl amide, *N,N-*diethyl-3-aminopropionic acid dimethylamine amide, *N*,*N*-diethyl-3-aminopropionic acid ethylamine amide, *N,N*-diethy(-3-aminopropionic acid diethylamine amide, *N,N-*diethyl-3-aminopropionic acid (*N*'-methyl-*N*'-ethylamine)amide, -*N,N*-diethyl-3-aminopropionic acid *n*-propylamine amide, *N*,*N*-diethyl-3-aminopropionic acid di-*n-*propylamine amide, *N*,*N*-diethyl-3-aminopropionic acid (*N*'-methyl-*N*'-*n-*propylamine)amide, *N*,*N*-diethyl-3-aminopropionic acid (*N*'-ethyl-*N*'-*n-*propylamine)amide, *N*,*N*-diethyl-3-aminopropionic acid *iso*-propylamine amide, *N*,*N-*diethyl-3-aminopropionic acid di-*iso*-propylamine amide, *N*,*N*-diethyl-3-aminopropionic acid (*N*'-methyl-*N*'-*iso*-propylamine)amide, *N,N-*diethyl-3-aminopropionic acid (*N*'-ethyl-*N*'-*iso*-propylamine)amide, *N*,*N*-diethyl-3-aminopropionic acid (*N*'-*n*-propyl-*N*'-*iso*-propylamine)amide,

*N*,*N*-diethyl-3-aminopropionic acid allylamine amide, *N*,*N*-diethyl-3-aminopropionic acid diallylamine amide, *N*,*N*-diethyl-3-aminopropionic acid (*N*'-methyl-*N*'-allylamine)amide, *N*,*N*-diethyl-3-aminopropionic acid (*N*'-ethyl-*N*'-allylamine)amide, *N*,*N*-diethyl-3-aminopropionic acid (*N*'-*n*-propyl-*N*'-allylamine)amide, *N,N-*diethyl-3-aminopropionic acid (*N*'-*iso*-propyl-*N*'-allylamine)amide, *N,N-*diethyl-3-aminopropionic acid cyclohexylamine amide, *N,N-*diethyl-3-aminopropionic acid piperidine amide, *N*,*N*-diethyl-3-aminopropionic acid pyrrolidine amide, *N*,*N*-diethyl-3-aminopropionic acid hexamethyleneimine amide, *N-*ethyl-*N*-methyl-3-aminopropionic acid amide, *N*-ethyl-*N*-methyl-3-aminopropionic acid methylamine amide, *N*-ethyl-*N*-methyl-3-aminopropionic acid dimethylamine amide, *N*-ethyl-*N*-methyl-3-aminopropionic acid ethylamine amide, *N*-ethyl-*N*-methyl-3-aminopropionic acid diethylamine amide, *N*-ethyl-*N*-methyl-3-aminopropionic acid (*N*'-methyl-*N*'-ethylamine)amide, *N*-ethyl-*N*-methyl-3-aminopropionic acid *n-*propylamine amide, *N*-ethyl-*N*-methyl-3-aminopropionic acid di-*n*-propylamine amide, *N*-ethyl-*N*-methyl-3-aminopropionic acid (*N*'-methyl-*N*'-*n*-propylamine)amide, *N*-ethyl-*N*-methyl-3-aminopropionic acid (*N*'-ethyl-*N*'-*n*-propylamine)amide, *N*-ethyl-*N-*methyl-3-aminopropionic acid *iso-*propylamine amide, *N*-ethyl-*N*-methyl-3-aminopropionic acid di-*iso*-propylamine amide,

*N*-ethyl-*N*-methyl-3-aminopropionic acid (*N*'-methyl-*N*'-iso-propylamine)amide, *N*-ethyl-*N*-methyl-3-aminopropionic acid (*N*'-ethyl-*N*'-*iso*-propylamine)amide, *N*-ethyl-*N*-methyl-3-aminopropionic acid (*N*'-*n*-propyl-*N*'-*iso*-propylamine)amide, *N*-ethyl-*N-*methyl-3-aminopropionic acid allylamine amide, *N*-ethyl-*N*-methyl-3-aminopropionic acid diallylamine amide, *N*-ethyl-*N*-methyl-3-aminopropionic acid (*N*'-methyl-*N*'-allylamine)amide, *N*-ethyl-*N*-methyl-3-aminopropionic acid (*N*'-ethyl-*N*'-allylamine)amide, *N*-ethyl-*N*-methyl-3-aminopropionic acid (*N*'-*n*-propyl-*N*'-allylamine)amide, *N*-ethyl-*N*-methyl-3-aminopropionic acid (*N*'-*iso*-propyl-*N*'-allylamine)amide, *N-*ethyl-*N*-methyl-3-aminopropionic acid cyclohexylamine amide, *N*-ethyl-*N*-methyl-3-aminopropionic acid piperidine amide, *N*-ethyl-*N*-methyl-3-aminopropionic acid pyrrolidine amide, *N*-ethyl-*N*-methyl-3-aminopropionic acid hexamethyleneimine amide, *N,N*-di(*n*-propyl)-3-aminopropionic acid amide, *N,N*-di(*n-*propyl)-3-aminopropionic acid methyl amide, *N,N*-di(*n*-propyl)-3-aminopropionic acid dimethylamine amide, *N,N*-di(*n*-propyl)-3-aminopropionic acid ethylamine amide, *N,N*-di(*n*-propyl)-3-aminopropionic acid diethylamine amide, *N*,*N*-di(*n*-propyl)-3-aminopropionic acid (*N*'-methyl-*N*'-ethylamine)amide, *N,N*-di(*n*-propyl)-3-aminopropionic acid *n*-propylamine amide, *N,N*-di(*n*-propyl)-3-aminopropionic acid di-*n*-propylamine amide,

*N,N*-di(*n*-propyl)-3-aminopropionic acid (*N*'-methyl-*N*'-*n*-propylamine)amide, *N,N*-di(*n*-propyl)-3-aminopropionic acid (*N*'-ethyl-*N*'-*n*-propylamine)amide, *N,N-*di(*n-*propyl)-3-aminopropionic acid *iso*-propylamine amide, *N*,*N*-di(*n*-propyl)-3-aminopropionic acid di-*iso*-propylamine amide, *N,N*-di(*n*-propyl)-3-aminopropionic acid (*N*'-methyl-*N*'-*iso*-propylamine)amide, *N,N*-di(*n*-propyl)-3-aminopropionic acid (*N*'-ethyl-*N*'-*iso*-propylamine)amide, *N,N*-di(*n*-propyl)-3-aminopropionic acid (*N*'-*n-*propyl-*N*'-*iso*-propylamine)amide, *N,N*-di(*n*-propyl)-3-aminopropionic acid allylamine amide, *N,N*-di(*n*-propyl)-3-aminopropionic acid diallylamine amide, *N*,*N*-di(*n*-propyl)-3-aminopropionic acid (*N*'-methyl-*N*'-allylamine)amide, *N*,*N*-di(*n*-propyl)-3-aminopropionic acid (*N*'-ethyl-*N*'-allylamine)amide, *N,N*-di(*n*-propyl)-3-aminopropionic acid (*N*'-*n*-propyl-*N*'-allylamine)amide, *N,N-*di(*n*-propyl)-3-aminopropionic acid (*N*'-*iso*-propyl-*N*'-allylamine)amide, *N*,*N*-di(*n*-propyl)-3-aminopropionic acid cyclohexylamine amide, *N,N*-di(*n*-propyl)-3-aminopropionic acid piperidine amide, *N,N*-di(*n-*propyl)-3-aminopropionic acid pyrrolidine amide, *N*,*N*-di(*n*-propyl)-3-aminopropionic acid hexamethyleneimine amide, *N*-*n*-propyl-*N*-methyl-3-aminopropionic acid amide, *N*-*n*-propyl-*N*-methyl-3-aminopropionic acid methyl amine amide,

*N-n*-propyl-*N*-methyl-3-aminopropionic acid dimethylamine amide, *N*-*n*-propyl-*N*-methyl-3-aminopropionic acid ethylamine amide, *N*-*n*-propyl-*N*-methyl-3-aminopropionic acid diethylamine amide, *N-n*-propyl-*N*-methyl-3-aminopropionic acid (*N*'-methyl-*N*'-ethylamine)amide, *N-n*-propyl-*N*-methyl-3-aminopropionic acid *n-*propylamine amide, *N-n*-propyl-*N*-methyl-3-aminopropionic acid di-*n*-propylamine amide, *N-n*-propyl-*N*-methyl-3-aminopropionic acid (*N*'-methyl-*N*'-*n-*propylamine)amide, *N-n*-propyl-*N*-methyl-3-aminopropionic acid (*N*'-ethyl-*N*'-*n-*propylamine)amide, *N-n*-propyl-*N-*methyl-3-aminopropionic acid *iso*-propylamine amide, *N-n*-propyl-*N*-methyl-3-aminopropionic acid di-*iso*-propylamine amide, *N*-*n-*propyl-*N*-methyl-3-aminopropionic acid (*N*'-methyl-*N*'-*iso*-propylamine)amide, *N-n-*propyl-*N*-methyl-3-aminopropionic acid (*N*'-ethyl-*N*'-*iso*-propylamine)amide, *N-n-*propyl-*N*-methyl-3-aminopropionic acid (*N*'-*n*-propyl-*N*'-*iso-*propylamine)amide, *N-n-*propyl-*N*-methyl-3-aminopropionic acid allylamine amide, *N*-*n*-propyl-*N*-methyl-3-aminopropionic acid diallylamine amide, *N-n*-propyl-*N*-methyl-3-aminopropionic acid (*N*'-methyl-*N*'-allylamine)amide, *N-n*-propyl-*N*-methyl-3-aminopropionic acid (*N*'-ethyl-*N*'-allylamine)amide, *N-n*-propyl-*N*-methyl-3-aminopropionic acid (*N*'-*n*-propyl-*N*'-allylamine)amide, *N-n*-propyl-*N*-methyl-3-aminopropionic acid (*N*'-*iso*-propyl-*N*'-allylamine)amide,

*N-n*-propyl-*N*-methyl-3-aminopropionic acid cyclohexylamine amide, *N*-*n-*propyl-*N*-methyl-3-aminopropionic acid piperidine amide, *N*-*n*-propyl-*N*-methyl-3-aminopropionic acid pyrrolidine amide, *N-n*-propyl-*N*-methyl-3-aminopropionic acid hexamethyleneimine amide, *N*-ethyl-*N-n*-propyl-3-aminopropionic acid amide, *N-*ethyl-*N*-*n*-propyl-3-aminopropionic acid methylamine amide, *N*-ethyl-*N-n*-propyl-3-aminopropionic acid dimethylamine amide, *N*-ethyl-*N-n*-propyl-3-aminopropionic acid ethylamine amide, *N*-ethyl-*N-n-*propyl-3-aminopropionic acid diethylamine amide, *N-*ethyl-*N-n-*propyl-3-aminopropionic acid (*N*'-methyl-*N*'-ethylamine)amide, *N*-ethyl-*N*-*n-*propyl-3-aminopropionic acid *n*-propylamine amide, *N*-ethyl-*N*-*n*-propyl-3-aminopropionic acid di-*n*-propylamine amide, *N*-ethyl-*N-n*-propyl-3-aminopropionic acid (*N*'-methyl-*N*'-*n*-propylamine)amide, *N*-ethyl-*N*-*n*-propyl-3-aminopropionic acid (*N*'-ethyl-*N*'-*n*-propylamine)amide, *N*-ethyl-*N*-*n*-propyl-3-aminopropionic acid *iso-*propylamine amide, *N*-ethyl-*N*-*n-*propyl-3-aminopropionic acid di-*iso*-propylamine amide, *N*-ethyl-*N*-*n*-propyl-3-aminopropionic acid (*N*'-methyl-*N*'*-iso-*propylamine)amide, *N*-ethyl-*N-n*-propyl aminopropionic acid (*N*'-ethyl-*N*'-*iso-*propylamine)amide, *N*-ethyl-*N*-*n*-propyl-3-aminopropionic acid (*N*'-*n*-propy-*N*'-*iso-*propylamine)amide, *N*-ethyl-*N-n-*propyl-3-aminopropionic acid allylamine amide,

*N*-ethyl-*N-n*-propyl-3-aminopropionic acid diallylamine amide, *N*-ethyl-*N*-*n-*propyl-3-aminopropionic acid (*N*'-methyl-*N*'-allylamine)amide, *N*-ethyl-*N*-*n*-propyl-3-aminopropionic acid (*N*'-ethyl-*N*'-allylamine)amide, *N*-ethyl-*N*-*n*-propyl-3-aminopropionic acid (*N*'-*n*-propyl-*N*'-allylamine)amide, *N*-ethyl-*N-n*-propyl-3-aminopropionic acid (*N*'-*iso*-propyl-*N*'-allylamine)amide, *N*-ethyl-*N*-*n*-propyl-3-aminopropionic acid cyclohexylamine amide, *N*-ethyl-*N-n*-propyl-3-aminopropionic acid piperidine amide, *N*-ethyl-*N-n*-propyl-3-aminopropionic acid pyrrolidine amide, *N*-ethyl-*N-n*-propyl-3-aminopropionic acid hexamethyleneimine amide, *N*,*N*-di(*iso-*propyl)-3-aminopropionic acid amide, *N,N*-di(*iso*-propyl)-3-aminopropionic acid methyl amide, *N,N*-di(*iso*-propyl)-3-aminopropionic acid dimethylamine amide, *N*,*N-*di(*iso*-propyl)-3-aminopropionic acid ethylamine amide, *N,N*-di(*iso*-propyl)-3-aminopropionic acid diethylamine amide, *N,N*-di(*iso*-propyl)-3-aminopropionic acid (*N*'-methyl-*N*'-ethylamine)amide, *N,N-*di(*iso*-propyl)-3-aminopropionic acid *n-*propylamine amide, *N,N*-di(*iso*-propyl)-3-aminopropionic acid di-*n*-propylamine amide, *N,N*-di(*iso*-propyl)-3-aminopropionic acid (*N*'-methyl-*N*'-*n*-propylamine)amide, *N,N*-di(*iso*-propyl)-3-aminopropionic acid (*N*'-ethyl-*N*'-*n*-propylamine)amide, *N,N-*di(*iso*-propyl)-3-aminopropionic acid *iso*-propylamine amide, *N*,*N*-di(*iso*-propyl)-3-aminopropionic acid di-*iso*-propylamine amide,

*N*,*N*-di(*iso*-propyl)-3-aminopropionic acid (*N*'-methyl-*N*'-*iso*-propylamine)amide, *N,N*-di(*iso*-propyl)-3-aminopropionic acid (*N*'-ethyl-*N*'-*iso*-propylamine)amide, *N*,*N-*di(*iso*-propyl)-3-aminopropionic acid (*N*'-*n*-propyl-*N*'-*iso*-propylamine)amide, *N*,*N-*di(*iso*-propyl)-3-aminopropionic acid allylamine amide, *N,N*-di(*iso*-propyl)-3-aminopropionic acid diallylamine amide, *N,N*-di(*iso*-propyl)-3-aminopropionic acid (*N*'-methyl-*N*'-allylamine)amide, *N,N*-di(*iso*-propyl)-3-aminopropionic acid (*N*'-ethyl-*N*'-allylamine)amide, *N,N*-di(*iso*-propyl)-3-aminopropionic acid (*N*'-*n*-propyl-*N*'-allylamine)amide, *N,N*-di(*iso*-propyl)-3-aminopropionic acid (*N*'-*iso*-propyl-*N*'-allylamine)amide, *N*,*N*-di(*iso*-propyl)-3-aminopropionic acid cyclohexylamine amide, *N,N*-di(*iso*-propyl)-3-aminopropionic acid piperidine amide, *N*,*N*-di(*iso*-propyl)-3-aminopropionic acid pyrrolidine amide, *N,N*-di(*iso*-propyl)-3-aminopropionic acid hexamethyleneimine amide, *N-iso*-propyl-*N-*methyl-3-aminopropionic acid amide, *N-iso*-propyl-*N*-methyl-3-aminopropionic acid methylamine amide, *N-iso*-propyl-*N-*methyl-3-aminopropionic acid dimethylamine amide, *N*-iso-propyl-*N*-methyl-3-aminopropionic acid ethylamine amide, *N-iso-*propyl-*N*-methyl-3-aminopropionic acid diethylamine amide,

*N-iso*-propyl-*N*-methyl-3-aminopropionic acid (*N*'-methyl-*N*'-ethylamine)amide, *N-iso*-propyl-*N*-methyl-3-aminopropionic acid *n*-propylamine amide, *N-iso-*propyl-*N-*methyl-3-aminopropionic acid di-*n*-propylamine amide, *N*-iso-propyl-N-methyl-3-aminopropionic acid (*N*'-methyl-*N*'-*n*-propylamine)amide, *N*-iso-propyl-N-methyl-3-aminopropionic acid (*N*'-ethyl-*N*'-*n*-propylamine)amide, *N*-iso-propyl-N-methyl-3-aminopropionic acid *iso*-propylamine amide, *N-iso*-propyl-*N*-methyl-3-aminopropionic acid di-*iso*-propylamine amide, *N-iso*-propyl-*N*-methyl-3-aminopropionic acid (*N*'-methyl-*N*'-*iso-*propylamine)amide, *N-iso*-propyl-*N*-methyl-3-aminopropionic acid (*N*'-ethyl-*N*'-*iso*-propylamine)amide, *N-iso*-propyl-*N*-methyl-3-aminopropionic acid (*N*'-*n-*propyl-*N*'-*iso*-propylamine)amide, *N-iso*-propyl-*N*-methyl-3-aminopropionic acid allylamine amide, *N-iso*-propyl-*N*-methyl-3-aminopropionic acid diallylamine amide, *N-iso-*propyl-*N*-methyl-3-aminopropionic acid (*N*'-methyl-*N*'-allylamine)amide, *N*-*iso-*propyl-*N*-methyl-3-aminopropionic acid (*N*'-ethyl-*N*'-allylamine)amide, *N*-*iso*-propyl-*N-*methyl-3-aminopropionic acid (*N*'-*n*-propyl-*N*'-allylamine)amide, *N*-*iso*-propyl-*N-*methyl-3-aminopropionic acid (*N*'-*iso*-propyl-*N*'-allylamine)amide, *N*-*iso*-propyl-*N-*methyl-3-aminopropionic acid cyclohexylamine amide,

*N-iso*-propyl-*N*-methyl-3-aminopropionic acid piperidine amide, *N-iso-*propyl-*N*-methyl-3-aminopropionic acid pyrrolidine amide, *N*-*iso*-propyl-*N*-methyl-3-aminopropionic acid hexamethyleneimine amide, *N*-ethyl-*N-iso*-propyl-3-aminopropionic acid amide, *N*-ethyl-*N-iso*-propyl-3-aminopropionic acid methylamine amide, *N*-ethyl-*N-iso*-propyl-3-aminopropionic acid dimethylamine amide, *N*-ethyl-*N-*iso-propyl-3-aminopropionic acid ethylamine amide, *N*-ethyl-*N*-*iso*-propyl-3-aminopropionic acid diethylamine amide, *N*-ethyl-*N-iso*-propyl-3-aminopropionic acid (*N*'-methyl-*N*-ethylamine)amide, *N*-ethyl-*N-iso-*propyl-3-aminopropionic acid *n-*propylamine amide, *N*-ethyl-*N-iso*-propyl-3-aminopropionic acid di-*n*-propylamine amide, *N*-ethyl-*N*-*iso*-propyl-3-aminopropionic acid (*N*'-methyl-*N*'-*n-*propylamine)amide, *N*-ethyl-*N-iso*-propyl-3-aminopropionic acid (*N*'-ethyl-*N*'-*n-*propylamine)amide, *N*-ethyl-*N*-*iso-*propyl-3-aminopropionic acid *iso-*propylamine amide, *N*-ethyl-*N-iso-*propyl-3-aminopropionic acid di*-iso*-propylamine amide, *N-*ethyl-*N*-*iso*-propyl-3-aminopropionic acid (*N*'-methyl-*N*'-*iso*-propylamine)amide, *N-*ethyl-*N*-*iso*-propyl-3-aminopropionic acid (*N*'-ethyl-*N*'-*iso*-propylamine)amide, *N-*ethyl-*N*-*iso*-propyl-3-aminopropionic acid (*N*'-*n*-propyl-*N*'-*iso*-propylamine)amide,

*N*-ethyl-*N-iso*-propyl-3-aminopropionic acid allylamine amide, *N*-ethyl-*N*-*iso-*propyl-3-aminopropionic acid diallylamine amide, *N*-ethyl-*N-iso*-propyl-3-aminopropionic acid (*N*'-methyl-*N*'-allylamine)amide, *N*-ethyl-*N*-*iso*-propyl-3-aminopropionic acid (*N*'-ethyl-*N*'-allylamine)amide, *N*-ethyl-*N*-*iso*-propyl-3-aminopropionic acid (*N*'-*n*-propyl-*N*'-allylamine)amide, *N*-ethyl-*N*-*iso*-propyl-3-aminopropionic acid (*N*'-*iso*-propyl-*N*'-allylamine)amide, *N*-ethyl-*N-iso*-propyl-3-aminopropionic acid cyclohexylamine amide, *N*-ethyl-*N-iso*-propyl-3-aminopropionic acid piperidine amide, *N*-ethyl-*N-iso*-propyl-3-aminopropionic acid pyrrolidine amide, *N*-ethyl-*N-iso-*propyl-3-aminopropionic acid hexamethyleneimine amide, *N-n-*propyl-*N*-*iso*-propyl-3-aminopropionic acid amide, *N-n-*propyl-*N-iso-*propyl-3-aminopropionic acid methylamine amide, *N-n-*propyl-*N-iso*-propyl-3-aminopropionic acid dimethylamine amide, *N-n*-propyl-*N-iso-*propyl-3-aminopropionic acid ethylamine amide, *N-n*-propyl-*N-iso*-propyl-3-aminopropionic acid diethylamine amide, *N*-*n-*propyl-*N*-*iso*-propyl-3-aminopropionic acid (*N*'-methyl-*N*'-ethylamine)amide, *N*-*n-*propyl-*N*-*iso*-propyl-3-aminopropionic acid *n*-propylamine amide, *N-n*-propyl-*N-iso-*propyl-3-aminopropionic acid di-*n*-propylamine amide, *N-n*-propyl-*N*-*iso*-propyl-3-aminopropionic acid (*N*'-methyl-*N*'-*n*-propylamine)amide, *N-n-*propyl-*N-iso*-propyl-3-aminopropionic acid (*N*'-ethyl-*N*'-*n*-propylamine)amide,

*N-n-*propyl-*N-iso*-propyl-3-aminopropionic acid *iso*-propylamine amide, *N*-*n-*propyl-*N*-*iso*-propyl-3-aminopropionic acid di-*iso*-propylamine amide, *N*-*n*-propyl-*N-iso*-propyl-3-aminopropionic acid (*N*'-methyl-*N*'-*iso*-propylamine)amide, *N-n*-propyl-*N-iso*-propyl-3-aminopropionic acid (*N*'-ethyl-*N*'-*iso*-propylamine)amide, *N*-*n*-propyl-*N-iso*-propyl-3-aminopropionic acid (*N*'-*n*-propyl-*N*'-*iso*-propylamine)amide, *N-n*-propyl-*N*-*iso*-propyl-3-aminopropionic acid allylamine amide, *N*-*n*-propyl-*N*-*iso*-propyl-3-aminopropionic acid diallylamine amide, *N*-*n*-propyl-*N*-*iso-*propyl-3-aminopropionic acid (*N*'-methyl-*N*'-allylamine)amide, *N-n*-propyl-*N-iso*-propyl-3-aminopropionic acid (*N*'-ethyl-*N*'-allylamine)amide, *N-n*-propyl-*N*-*iso*-propyl-3-aminopropionic acid (*N*'-*n-*propyl-*N*'-allylamine)amide, *N-n-*propyl-*N-iso*-propyl 3-aminopropionic acid (*N*'-*iso-*propyl-*N*'-allylamine)amide, *N-n-*propyl-*N*-*iso*-propyl-3-aminopropionic acid cyclohexylamine amide, *N-n-*propyl-*N-iso*-propyl-3-aminopropionic acid piperidine amide, *N-n-*propyl-*N-iso*-propyl-3-aminopropionic acid pyrrolidine amide, *N-n-*propyl-*N*-*iso*-propyl-3-aminopropionic acid hexamethyleneimine amide, *N*,*N*-diallyl-3-aminopropionic acid amide, *N,N-*diallyl-3-aminopropionic acid methyl amide, *N*,*N-*diallyl-3-aminopropionic acid dimethylamine amide,

*N,N-*diallyl-3-aminopropionic acid ethylamine amide, *N,N-*diallyl-3-aminopropionic acid diethylamine amide, *N*,*N*-diallyl-3-aminopropionic acid (*N-*methyl-*N*'-ethylamine)amide, *N,N*-diallyl-3-aminopropionic acid *n*-propylamine amide, *N,N*-diallyl-3-aminopropionic acid di-*n*-propylamine amide, *N*,*N*-diallyl-3-aminopropionic acid (*N*'-methyl-*N*'-*n*-propylamine)amide, *N,N*-diallyl-3-aminopropionic acid (*N*'-ethyl-*N*'-*n*-propylamine)amide, *N,N-*diallyl-3-aminopropionic acid *iso*-propylamine amide, *N,N*-diallyl-3-aminopropionic acid di-*iso*-propylamine amide, *N*,*N*-diallyl-3-aminopropionic acid (*N*'-methyl-*N*'-*iso*-propylamine)amide, *N,N-*diallyl-3-aminopropionic acid (*N*'-ethyl-*N*'-*iso*-propylamine)amide, *N*,*N*-diallyl-3-aminopropionic acid (*N*'-*n*-propyl-*N*'-*iso*-propylamine)amide, *N*,*N*-diallyl-3-aminopropionic acid allylamine amide, *N*,*N*-diallyl-3-aminopropionic acid diallylamine amide, *N,N*-diallyl-3-aminopropionic acid (*N*'-methyl-*N*'-allylamine)amide, *N*,*N*-diallyl-3-aminopropionic acid (*N*'-ethyl-*N*'-allylamine)amide, *N,N*-diallyl-3-aminopropionic acid (*N*'-*n*-propyl-*N*'-allylamine)amide, *N,N-*diallyl-3-aminopropionic acid (*N*'-*iso-*propyl-*N*'-allylamine)amide, *N*,*N*-diallyl-3-aminopropionic acid cyclohexylamine amide, *N*,*N*-diallyl-3-aminopropionic acid piperidine amide, *N*,*N*-diallyl-3-aminopropionic acid pyrrolidine amide, *N,N*-diallyl-3-aminopropionic acid hexamethyleneimine amide,

*N*-allyl-*N*-methyl-3-aminopropionic acid amide, *N*-allyl-*N*-methyl-3-aminopropionic acid methylamine amide, *N*-allyl-*N*-methyl-3-aminopropionic acid dimethylamine amide, *N*-allyl-*N*-methyl-3-aminopropionic acid ethylamine amide, *N-*allyl-*N*-methyl-3-aminopropionic acid diethylamine amide, *N*-allyl-*N*-methyl-3-aminopropionic acid (*N*'-methyl-*N*'-ethylamine)amide, *N*-allyl-*N*-methyl-3-aminopropionic acid *n*-propylamine amide, *N*-allyl-*N*-methyl-3-aminopropionic acid di-*n*-propylamine amide, *N*-allyl-*N*-methyl-3-aminopropionic acid (*N*'-methyl-*N*'-*n-*propylamine)amide, *N*-allyl-*N*-methyl-3-aminopropionic acid (*N*'-ethyl-*N*'-*n-*propylamine)amide, *N*-allyl-*N*-methyl-3-aminopropionic acid *iso-*propylamine amide, *N*-allyl-*N*-methyl-3-aminopropionic acid di-*iso*-propylamine amide, *N*-allyl-*N*-methyl-3-aminopropionic acid (*N*'-methyl-*N*'-*iso*-propylamine)amide, *N*-allyl-*N*-methyl-3-aminopropionic acid (*N*'-ethyl-*N*'-*iso*-propylamine)amide, *N*-allyl-*N*-methyl-3-aminopropionic acid (*N*'-*n*-propyl-*N*'-*iso*-propylamine)amide, *N*-allyl-*N*-methyl-3-aminopropionic acid allylamine amide, *N*-allyl-*N*-methyl-3-aminopropionic acid diallylamine amide, *N*-allyl-*N*-methyl-3-aminopropionic acid (*N*-methyl-*N*'-allylamine)amide, *N*-allyl-*N*-methyl-3-aminopropionic acid (*N*'-ethyl-*N*'-allylamine)amide, *N*-allyl-*N*-methyl-3-aminopropionic acid (*N*'-*n*-propyl-*N*'-allylamine)amide, *N*-allyl-*N*-methyl-3-aminopropionic acid (*N*'-*iso-*propyl-*N*'-allylamine)amide,

*N*-allyl-*N*-methyl-3-aminopropionic acid cyclohexylamine amide, *N*-allyl-*N-*methyl-3-aminopropionic acid piperidine amide, *N*-allyl-*N*-methyl-3-aminopropionic acid pyrrolidine amide, *N*-allyl-*N*-methyl-3-aminopropionic acid hexamethyleneimine amide, *N*-allyl-*N*-ethyl-3-aminopropionic acid amide, *N*-allyl-*N*-ethyl-3-aminopropionic acid methylamine amide, *N*-allyl-*N*-ethyl-3-aminopropionic acid dimethylamine amide, *N*-allyl-*N*-ethyl-3-aminopropionic acid ethylamine amide, *N*-allyl-*N*-ethyl-3-aminopropionic acid diethylamine amide, *N*-allyl-*N*-ethyl-3-aminopropionic acid (*N*'-methyl-*N*'-ethylamine)amide, *N*-allyl-*N*-ethyl-3-aminopropionic acid *n*-propylamine amide, *N*-allyl-*N*-ethyl-3-aminopropionic acid di-*n*-propylamine amide, *N*-allyl-*N*-ethyl-3-aminopropionic acid (*N*'-methyl-*N*'-*n*-propylamine)amide, *N*-allyl-*N*-ethyl-3-aminopropionic acid (*N*'-ethyl-*N*'-*n*-propylamine)amide, *N*-allyl-*N*-ethyl-3-aminopropionic acid *iso*-propylamine amide, *N*-allyl-*N*-ethyl-3-aminopropionic acid di-*iso*-propylamine amide, *N*-allyl-*N*-ethyl-3-aminopropionic acid (*N*'-methyl-*N*'*-iso-*propylamine)amide, *N*-allyl-*N*-ethyl-3-aminopropionic acid (*N*'-ethyl-*N*'-*iso-*propylamine)amide, *N*-allyl-*N*-ethyl-3-aminopropionic acid (*N*'-*n*-propyl-*N*'-*iso-*propylamine)amide, *N*-allyl-*N*-ethyl-3-aminopropionic acid allylamine amide, *N*-allyl-*N*-ethyl-3-aminopropionic acid diallylamine amide,

*N*-allyl-*N-*ethyl-3-aminopropionic acid (*N*'-methyl-*N*'-allylamine)amide, *N*-allyl-*N*-ethyl-3-aminopropionic acid (*N*'-ethyl-*N*'-allylamine)amide, *N*-allyl-*N*-ethyl-3-aminopropionic acid (*N*'-*n*-propyl-*N*'-allylamine)amide, *N*-allyl-*N*-ethyl-3-aminopropionic acid (*N*'-*iso*-propyl-*N*-allylamine)amide, *N*-allyl-*N*-ethyl-3-aminopropionic acid cyclohexylamine amide, *N*-allyl-*N*-ethyl-3-aminopropionic acid piperidine amide, *N*-allyl-*N*-ethyl-3-aminopropionic acid pyrrolidine amide, *N*-allyl-*N-*ethyl-3-aminopropionic acid hexamethyleneimine amide, *N*-allyl-*N*-*n*-propyl-3-aminopropionic acid amide, *N*-allyl-*N-n-*propyl-3-aminopropionic acid methylamine amide, *N*-allyl-*N-n*-propyl-3-aminopropionic acid dimethylamine amide, *N*-allyl-*N-n-*propyl-3-aminopropionic acid ethylamine amide, *N*-allyl-*N-n*-propyl-3-aminopropionic acid diethylamine amide, *N*-allyl-*N-n*-propyl-3-aminopropionic acid (*N*'-methyl-*N*'-ethylamine)amide, *N*-allyl-*N-n*-propyl-3-aminopropionic acid *n*-propylamine amide, *N-*allyl-*N-n*-propyl-3-aminopropionic acid di-*n*-propylamine amide, *N*-allyl-*N*-*n*-propyl-3-aminopropionic acid (*N*'-methyl-*N*'-*n*-propylamine)amide, *N*-allyl-*N*-*n*-propyl-3-aminopropionic acid (*N*'-ethyl-*N*'-*n*-propylamine)amide, *N*-allyl-*N*-*n*-propyl-3-aminopropionic acid *iso*-propylamine amide, *N*-allyl-*N-n*-propyl -3-aminopropionic acid di-*iso-*propylamine amide, *N*-allyl-*N-n*-propyl-3-aminopropionic acid (*N*'-methyl-*N*'-*iso*-propylamine)amide,

*N*-allyl-*N-n*-propyl-3-aminopropionic acid (*N*'-ethyl-*N*'-*iso*-propylamine)amide, *N*-allyl-*N-n*-propyl-3-aminopropionic acid (*N*'-*n*-propyl-*N*'-*iso*-propylamine)amide, *N-*allyl-*N-n*-propyl-3-aminopropionic acid allylamine amide, *N*-allyl-*N-n*-propyl-3-aminopropionic acid diallylamine amide, *N*-allyl-*N-n-*propyl-3-aminopropionic acid (*N*'-methyl-*N*'-allylamine)amide, *N*-allyl-*N-n*-propyl-3-aminopropionic acid (*N*'-ethyl-*N*'-allylamine)amide, *N*-allyl-*N-n*-propyl-3-aminopropionic acid (*N*'-*n*-propyl-*N*'-allylamine)amide, *N*-allyl-*N-n*-propyl-3-aminopropionic acid (*N*'-*iso-*propyl-*N*'-allylamine)amide, *N*-allyl-*N*-*n*-propyl-3-aminopropionic acid cyclohexylamine amide, *N*-allyl-*N-n*-propyl-3-aminopropionic acid piperidine amide, *N*-allyl-*N-n*-propyl-3-aminopropionic acid pyrrolidine amide, *N*-allyl-*N-n*-propyl-3-aminopropionic acid hexamethyleneimine amide, *N*-allyl-*N-iso*-propyl-3-aminopropionic acid amide, N-allyl-*N-iso*-propyl-3-aminopropionic acid methylamine amide, *N*-allyl-*N-iso*-propyl-3-aminopropionic acid dimethylamine amide, *N*-allyl-*N-iso*-propyl-3-aminopropionic acid ethylamine amide, *N*-allyl-*N-iso-*propyl-3-aminopropionic acid diethylamine amide, *N*-allyl-*N-iso-*propyl-3-aminopropionic acid (*N*'-methyl-*N*'-ethylamine)amide,

*N*-allyl-*N*-*iso*-propyl-3-aminopropionic acid *n*-propylamine amide, *N*-allyl-*N*-iso-propyl-3-aminopropionic acid di-*n*-propylamine amide, *N*-allyl-*N*-iso-propyl-3-aminopropionic acid (*N*'-methyl-*N'*-*n*-propylamine)amide, *N*-allyl-*N*-iso-propyl-3-aminopropionic acid (*N*'-ethyl-*N*'-n-propylamine)amide, *N*-allyl-*N*-iso-propyl-3-aminopropionic acid *iso*-propylamine amide, *N*-allyl-*N*-*iso*-propyl-3-aminopropionic acid di-*iso*-propylamine amide, *N*-allyl-*N*-*iso*-propyl-3-aminopropionic acid (*N*'-methyl-*N*'-*iso*-propylamine)amide, *N*-allyl-*N*-*iso*-propyl-3-aminopropionic acid (*N*'-ethyl-*N*'-*iso*-propylamine)amide, *N*-allyl-*N*-*iso*-propyl-3-aminopropionic acid (*N*'-n-propyl-*N*'-*iso*-propylamine)amide, *N*-allyl-*N*-*iso*-propyl-3-aminopropionic acid allylamine amide, *N*-allyl-*N*-*iso*-propyl-3-aminopropionic acid diallylamine amide, *N*-allyl-*N*-*iso*-propyl-3-aminopropionic acid (*N*'-methyl-*N*'-allylamine)amide, *N*-allyl-*N*-*iso*-propyl-3-aminopropionic acid (*N*'-ethyl-*N*'-allylamine)amide, *N*-allyl-*N*-*iso*-propyl-3-aminopropionic acid (*N*'-n-propyl-*N*-allylamine)amide, *N*-allyl-*N*-*iso*-propyl-3-aminopropionic acid (*N*'-*iso*-propyl-*N*'-allylamine)amide, *N*-allyl-*N*-*iso*-propyl-3-aminopropionic acid cyclohexylamine amide, *N*-allyl-*N*-*iso*-propyl-3-aminopropionic acid piperidine amide, *N*-allyl-*N*-*iso*-propyl-3-aminopropionic acid pyrrolidine amide, and *N*-allyl-*N*-*iso*-propyl-3-aminopropionic acid hexamethyleneimine amide.

β-alanine amide and β-alanine methylamide are preferred because the effect of the present invention will be good.

β-alanine derivatives represented by general formula (3) according to the present invention will now be described.

In general formula (3), R₅ and R₆ are each independently a hydrogen atom, C₁₋₇ alkyl group, C₁₋₇ alkenyl group, C₃₋₇ hydrocarbon group having a cyclic segment, pyridine group, cyclohexylcarbonyl group, cyclopentyl carbonyl group, nicotinoyl group, isonicotinoyl group, picolinoyl group, nipecotinoyl group, isonipecotinoyl group, N-acetyl nipecotinoyl group, N-acetyl isonipecotinoyl group, benzyloxycarbonyl group, group represented by general formula (4)

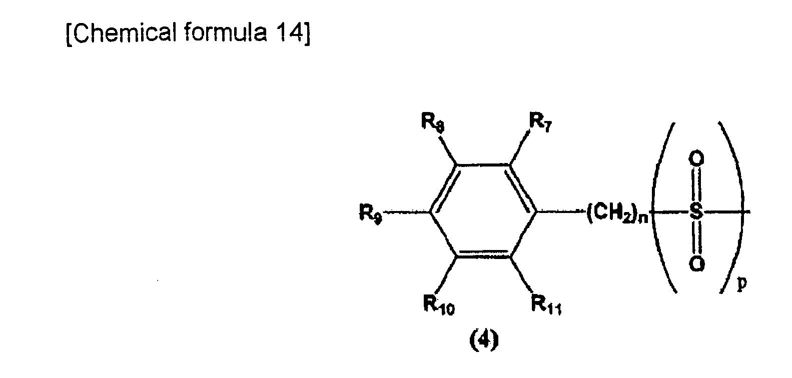

or a group represented by general formula (5)

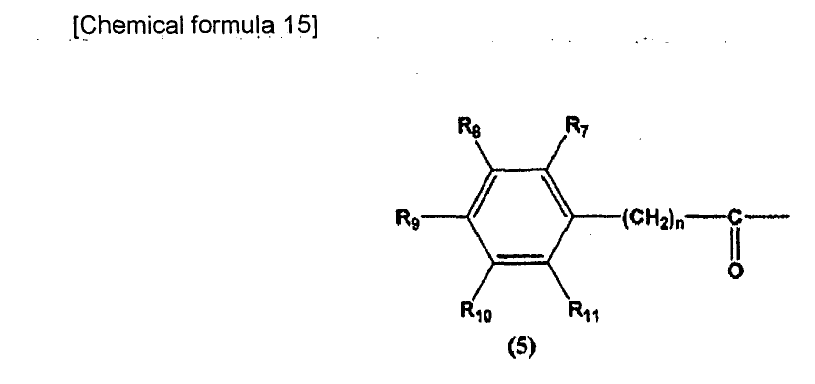

When one of R₅ and R₆ is a hydrogen atom, the other does not represent a hydrogen atom because R₅ and R₆ cannot both be hydrogen atoms.

Each group represented by R₅ and R₆ is described in detail below.

The C₁₋₇ alkyl group may be, e.g., a linear aliphatic chain or a branched aliphatic chain. The C₁₋₇ alkenyl group may also be, e.g., a linear aliphatic chain or a branched aliphatic chain.

Specific examples of C₁₋₇ alkyl groups and C₁₋₇ alkenyl groups are methyl, ethyl, *n*-propyl, *iso*-propyl, *n*-butyl, *iso*-butyl, *ter*-butyl, 1-methylpropyl, n-pentyl, 1-methylbutyl, 2-methylbutyl, 3-methylbutyl, 1,1-dimethylpropyl, 2,2-dimethylpropyl, 1,2-dimethylpropyl, 1-ethylpropyl, *n*-hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methyl-propyl, 1-ethyl-2-methylpropyl, n-heptyl, 1-methylhexyl, 2-methylhexyl, 3-methylhexyl, 4-methylhexyl, 5-methylhexyl, 1,1-dimethylpentyl, 1,2-dimethylpentyl, 1,3-dimethylpentyl, 1,4-dimethylpentyl, 2,2-dimethylpentyl, 2,3-dimethylpentyl, 2,4-dimethylpentyl, 3,3-dimethylpentyl, 3,4-dimethylpentyl,

4,4-dimethylpentyl, 1-ethylpentyl, 2-ethylpentyl, 3-ethylpentyl, 1,1,2-trimethylbutyl, 1,1,3-trimethylbutyl, 1,2,2-trimethylbutyl, 1,2,3-trimethylbutyl, 2,2,3-trimethylbutyl, 2,3,3-trimethylbutyl, 1-ethyl-1-methylbutyl, 1-ethyl-2-methylbutyl, 1-ethyl-3-methylbutyl, 2-ethyl-1-methylbutyl, 2-ethyl-2-methylbutyl, 2-ethyl-3-methylbutyl, 1-propylbutyl, 1-ethyl-1,2-dimethylbutyl, 1-ethyl-2,2-dimethylbutyl, 1-propyl-2-methylpropyl, 1-(1'-methylethyl)-2-methylbutyl, 1,1,2,2-tetramethylpropyl, allyl, 1-methylallyl, 2-butenyl, 3-butenyl, 3-methyl-2-butenyl, 2-pentenyl, 3-pentenyl, 4-pentenyl, 1,1-dimethylallyl, 1-methyl-2-butenyl, 1-methyl-3-butenyl, 2-hexenyl, 3-hexenyl, 4-hexenyl, 5-hexenyl, 2,4-hexadienyl, 4-methyl-3-pentenyl, 2-heptenyl, 3-heptenyl, 4-heptenyl, 5-heptenyl, 6-heptenyl, 2,4-heptadienyl, 1-methyl-4-hexenyl, and 5-methyl-4-hexenyl.

The C₃₋₇ hydrocarbon group having a cyclic segment can be a cyclic aliphatic chain, an aliphatic chain having a cyclic segment, an unsaturated aliphatic chain, or the like.

Specific examples of C₃₋₇ hydrocarbon groups having a cyclic segment are cyclopropyl, cyclopropylmethyl, 1-methylcyclopropyl, 2-methylcyclopropyl, 3-methylcyclopropyl, 1-cyclopropylethyl, 2-cyclopropylethyl, 1,2-dimethylcyclopropyl, 2,2-dimethylcyclopropyl, 2,3-dimethylcyclopropyl, 1-cyclopropylpropyl, 2-cyclopropylpropyl, 3-cyclopropylpropyl, (2'-methylcyclopropyl)methyl, (2'-ethylcyclopropyl)methyl, 1-ethylcyclopropyl, 2-ethylcyclopropyl, 1,2,3-trimethylcyclopropyl, 2,2,3-trimethylcyclopropyl, 1,2,2-trimethylcyclopropyl, (1',2'-dimethylcyclopropyl)methyl, (2',2'-dimethylcyclopropyl)methyl, (2', 3'-dimethylcyclopropyl)methyl, 1-(1'-methylcyclopropyl)ethyl, 1-(2'-methylcyclopropyl)ethyl, 2-cyclopropylpropyl, 2-(2'-methylcyclopropyl)ethyl, 1-(1'-methylcyclopropyl)propyl, 1-(2'-methylcyclopropyl)propyl, 2-ethyl-1-methylcyclopropyl, 2-ethyl-2-methylcyclopropyl, 3-cyclopropylbutyl, 2-cyclopropylbutyl, 1-cyclopropylbutyl, 2-cyclopropyl-1-methylethyl, 3-(2'-methylcyclopropyl)propyl, 1-(2'-propylcyclopropyl)methyl, 3-cyclopropyl-2-methylpropyl, 3-cyclopropyl-1-methylpropyl, 3-cyclopropylpentyl, 3-(2'-ethylcyclopropyl)ethyl, 1,2-diethylcyclopropyl, 2,2-diethylcyclopropyl,

2-cyclopropyl-1-ethylethyl, 2-(2',2'-dimethylcyclopropyl)ethyl, 2-(2',3'-dimethylcyclopropyl)ethyl, 2-(2', 2'-dimethylcyclopropyl)butyl, 2-(2',3'-dimethylcyclopropyl)butyl, 2'(2'-methylcyclopropyl)-1-methylethyl, 2-cyclopropyl-1-methylbutyl, tetramethylcyclopropyl, (2',2',3'-trimethylcyclopropyl)methyl, 1-(2',2'-dimethylcyclopropyl)ethyl, 1-(2',3'-dimethylcyclopropyl)ethyl, 1-(2',2'-dimethylcyclopropyl)-1-methylmethyl, 1-(2',3'-dimethylcyclopropyl)-1-methylmethyl, 1-(3'-methylcyclopropyl)-1-methylethyl, 1-(2'-methylcyclopropyl)-1-methylethyl, 1-(2'-ethylcyclopropyl)-1-methylmethyl, 1-cyclopropyl-1-methylethyl, cyclobutyl, cyclobutylmethyl, 1-methylcyclobutyl, 2-methylcyclobutyl, 3-methylcyclobutyl, 1,2-dimethylcyclobutyl, 1,3-dimethylcyclobutyl, 2,2-dimethylcyclobutyl, 2,3-dimethylcyclobutyl, 2,4-dimethylcyclobutyl, 3,3-dimethylcyclobutyl, 1-ethylcyclobutyl, 2-ethylcyclobutyl, 3-ethylcyclobutyl, 1-cyclobutylethyl, 2-cyclobutylethyl, 1-cyclobutyl-1-methylmethyl, (2,2'-dimethylcyclobutyl)methyl, (2',3'-dimethylcyclobutyl)methyl, 3',3'-dimethylcyclobutyl)methyl, (2',4'-dimethylcyclobutyl)methyl, 1-(2'-methylcyclobutyl)ethyl, 1-(3'-methylcyclobutyl)ethyl,

1,2,2-trimethylcyclobutyl, 1,2,3-trimethylcyclobutyl, 1,3,3-trimethylcyclobutyl, 1,2,4-trimethylcyclobutyl, 2,2,3-trimethylcyclobutyl, 2,3,3-trimethylcyclobutyl, 2,3,4-trimethylcyclobutyl, (2'-ethylcyclobutyl)methyl, (3'-ethylcyclobutyl)methyl, 1-cyclobutylpropyl, 2-cyclobutylpropyl, 3-cyclobutylpropyl, 1-propylcyclobutyl, 2-propylcyclobutyl, 1-ethyl-2-methylcyclobutyl, 2-ethyl-2-methylcyclobutyl, 1-ethyl-3-methylcyclobutyl, 3-ethyl-3-methylcyclobutyl, 2-ethyl-3-methylcyclobutyl, 3-ethyl-2-methylcyclobutyl, 2-ethyl-1-methylcyclobutyl, 3-ethyl-1-methylcyclobutyl, 2-ethyl-4-methylcyclobutyl, 4-ethyl-2-methylcyclobutyl 2-cyclobutyl-1-methylethyl, 2-(2'-methylcyclobutyl)ethyl, 2-(3'-methylcyclobutyl)ethyl, cyclopentyl, 1-methyl-2-propylcyclobutyl, 1-methyl-3-propylcyclobutyl, 2-methyl-2-propylcyclobutyl, 3-methyl-3-propylcyclobutyl, 2-methyl-3-propylcyclobutyl, 2-methyl-1-propylcyclobutyl, 3-methyl-1-propylcyclobutyl, 2-methyl-4-propylcyclobutyl, cyclopentyl, cyclopentylmethyl, (2'-methylcyclopentyl)methyl, (3'-methylcyclopentyl)methyl, 1-cyclopentylethyl, 2-cyclopentylethyl, 1,2-dimethylcyclopentyl, 2,2-dimethylcyclopentyl, 2,3-dimethylcyclopentyl,

3,3-dimethylcyclopentyl, 1,3-dimethylcyclopentyl, 2,5-dimethylcyclopentyl, 2,4-dimethylcyclopentyl, 3,5-dimethylcyclopentyl, 3,4-dimethylcyclopentyl, 1-cyclopentyl-1-methylmethyl, cyclohexylmethyl, 1-methylcyclohexyl, 2-methylcyclohexyl, 3-methylcyclohexyl, cycloheptyl, cyclopropylenyl, cyclopropylenylmethyl, cyclobutenyl, cyclobutadienyl, 1-cyclopentenyl, 2-cyclopentenyl, 3-cyclopentenyl, cyclopentadienyl, 2-methylcyclopentadienyl, 3-methylcyclopentadienyl, 2,2-dimethyl-1-cyclopentenyl, 2,3-dimethyl-1-cyclopentenyl, 2,4-dimethyl-1-cyclopentenyl, 2,5-dimethyl-1-cyclopentenyl, 3,3-dimethyl-1-cyclopentenyl, 3,4-dimethyl-1-cyclopentenyl, 3,5-dimethyl-1-cyclopentenyl, 4,4-dimethyl-1-cyclopentenyl, 4,5-dimethyl-1-cyclentenyl, 5,5-dimethyl-1-cyclopentenyl, 1,2-dimethyl-2-cyclopentenyl, 1,3-dimethyl-2-cyclopentenyl, 1,4-dimethyl-2-cyclopentenyl, 1,5-dimethyl-2-cyclopentenyl, 2,3-dimethyl-2-cyclopentenyl, 2,4-dimethyl-2-cyclopentenyl, 2,5-dimethyl-2-cyclentenyl, dimethylcyclopentadienyl, 3,4-dimethyl-2-cyclopentenyl, 3,5-dimethyl-2-cyclopentenyl, 4,4-dimethyl-2-cyclopentenyl, 4,5-dimethyl-2-cyclopentenyl, 5,5-dimethyl-2-cyclopentenyl,

cyclopentenylmethyl, 1,2-dimethyl-3-cyclopentenyl, 1,3-dimethyl-3-cyclopentenyl, 2,2-dimethyl-3-cyclopentenyl, 2,3-dimethyl-3-cyclopentenyl, 2,4-dimethyl-3-cyclopentenyl, 2,5-dimethyl-3-cyclopentenyl, 3,4-dimethyl-3-cyclopentenyl, 3,5-dimethyl-3-cyclopentenyl, 1-(1'-cyclopentenyl)ethyl, 1-(2'-cyclopentenyl)ethyl, 1-(3'-cyclopentenyl)ethyl, 2-(1'-cyclopentenyl)ethyl, 2-(2'-cyclopentenyl)ethyl, 2-(3'-cyclopentenyl)ethyl, 1-cyclohexenyl, 2-cyclohexenyl, 3-cyclohexenyl, 1,3-cyclohexadienyl, 1,4-cyclohexadienyl, 1,5-cyclohexadienyl, 2,4-cyclohexadienyl, 2,5-cyclohexadienyl, 2-methyl-1-cyclohexenyl, 3-methyl-1-cyclohexenyl, 4-methyl-1-cyclohexenyl,

5-methyl-1-cyclohexenyl, 6-methyl-1-cyclohexenyl, 1-methyl-2-cyclohexenyl, 2-methyl-2-cyclohexenyl, 3-methyl-2-cyclohexenyl, 4-methyl-2-cyclohexenyl, 5-methyl-2-cyclohexenyl, 6-methyl-2-cyclohexenyl, 1-methyl-3-cyclohexenyl, 2-methyl-3-cyclohexenyl, 3-methyl-3-cyclohexenyl, 4-methyl-3-cyclohexenyl, 5-methyl-3-cyclohexenyl 6-methyl-3-cyclohexenyl, 1-cycloheptenyl, 2-cycloheptenyl, 3-cycloheptenyl, 4-cycloheptenyl, (1'-cyclohexenyl)methyl, (2'-cyclohexenyl)methyl, (3'-cyclohexenyl)methyl, (1',3'-cyclohexadienyl)methyl, (1', 4'-cyclohexadienyl)methyl, (1',5'-cyclohexadienyl)methyl, (2', 4'-cyclohexadienyl)methyl, and (2,5'-cyclohexadienyl) methyl groups, but the present invention is not limited to these groups.

Cyclopropylmethyl, 2-cyclopropylethyl, 3-cyclopropylpropyl, 4-cyclopropylbutyl, cyclobutyl, cyclobutylmethyl, 2-cyclobutylethyl, 3-cyclobutylpropyl, cyclopentyl, cyclopentylmethyl, 2-cyclopentylethyl, cyclohexyl, cyclohexylmethyl, methylcyclohexyl, cycloheptyl, cyclohexenyl, and cyclohexadienyl groups are preferred because the synthesis in the present invention will be facilitated. Hydrocarbon groups having a cyclohexyl segment are the most preferred because the effect of the present invention will be good. Examples are cyclohexyl, cyclohexylmethyl, methylcyclohexyl, cyclohexenyl, and cyclohexadienyl groups.

In the general formula (4), p represents an integer of 0 or 1. When p is 0, the compound of the present invention is the *N*-allyl-β-alanine or *N*-aralkyl-β-alanine corresponding to R₇-R₁₁ and n. When p is 1, the compound of the present invention is the *N*-sulfonyl-β-alanine derivative corresponding to R₇-R₁₁ and n.

In the general formula (4), n represents an integer of 0, 1, or 2. In cases in which p is 0, the compound of the present invention is an *N*-phenyl-β-alanine derivative corresponding to R₇ through R₁₁ when n is 0. When n is 1, the compound of the present invention is an *N*-benzyl-β-alanine derivative corresponding to R₇ through R₁₁, and when n is 2, the compound of the present invention an *N-*phenylethyl-β-alanine derivative corresponding to R₇ through R₁₁. In cases in which p is 1, the compound of the present invention is an *N*-benzenesulfonyl-β-alanine derivative corresponding to R₇ through R₁₁ when n is 0. When n is 1, the compound of the present invention is an *N*-benzylsulfonyl-β-alanine derivative corresponding to R₇ through R₁₁, and when n is 2, the compound of the present invention is an *N-*phenylethylsulfonyl-β-alanine derivative corresponding to R₇ through R₁₁.

Moreover, in general formula (4), R₇ through R₁₁ each independently represent a hydrogen atom, hydroxyl group, C₁₋₄ alkyloxy group, C₁₋₄ alkenyloxy group, C₁₋₃ alkyl group, or C₁₋₃ alkenyl group. When the number of carbon atoms in the alkyloxy or alkenyloxy groups exceeds 4, the effect of the present invention is inferior because solubility in a pharmaceutical preparation is poor the molecular weight increases at the same time. Any alkyloxy and alkenyloxy group can be used as long as the hydrocarbon segment is a C₁₋₄ segment, and specific examples are methyl, ethyl, *n*-propyl, *iso*-propyl, *n*-butyl, *iso*-butyl, *tert*-butyl, 1-methylpropyl, allyl(2-propenyl), 2-butenyl, 3-butenyl, 1-methyl-2-propenyl, 2-methyl-2-propenyl, cyclopropyl, cyclobutyl, cyclopropylmethyl, cyclopropenyl, cyclopropenylmethyl, and cyclobutenyl groups.

When the number of carbon atoms in the alkyl and alkenyl group exceeds 3, the effect of the present invention will be inferior because solubility in a pharmaceutical preparation is poor and the molecular weight increases at the same time. Any alkyl or alkenyl group can be used as long as the hydrocarbon segment is a C₁₋₃ segment. Specific examples are methyl, ethyl, *n*-propyl, *iso*-propyl, allyl(2-propenyl), cyclopropyl, and cyclopropenyl groups. However, the present invention is not limited to these examples.

In terms of ease of compound synthesis, it is preferred that R₇ through R₁₁ in the general formula (4) each independently be a hydrogen, hydroxyl group, or C₁₋₄ alkyloxy group. It is preferred that each of R₇ through R₁₁ be a hydrogen atom, or that one or more of R₇ through R₁₁ be a C₁₋₄ alkyloxy group because the effect of the present invention will be good. It is preferred that the C₁₋₄ alkyloxy group be a methoxy group because solubility will be good.

Specific examples of groups represented by general formula (4) are phenyl, 4-methoxyphenyl, 3-methoxyphenyl, 2-methoxyphenyl, 4-ethoxyphenyl, 3-ethoxyphenyl, 2-ethoxyphenyl, 4-propoxyphenyl, 3-propoxyphenyl, 2-propoxyphenyl, 4-butoxyphenyl, 3-butoxyphenyl, 2-butoxyphenyl, 2,4-dimethoxyphenyl, 3,4-dimethoxyphenyl, 3,4,5-trimethoxyphenyl, 2-hydroxyphenyl, 3-hydroxyphenyl, 4-hydroxyphenyl, 2,4-dihydroxyphenyl, 3,4-dihydroxyphenyl, 3,4,5-trihydroxyphenyl, 2-hydroxy-4-methoxyphenyl, 3-hydroxy-4-methoxyphenyl, 4-hydroxy-3-methoxyphenyl, 2-methylphenyl, 3-methylphenyl, 4-methylphenyl, 2-ethylphenyl, 3-ethylphenyl, 4-ethylphenyl, 2-propylphenyl, 3-propylphenyl, 4-propylphenyl,

benzyl, 4-methoxybenzyl, 3-methoxybenzyl, 2-methoxybenzyl, 4-ethoxybenzyl, 3-ethoxybenzyl, 2-ethoxybenzyl, 4-propoxybenzyl, 3-propoxybenzyl, 2-propoxybenzyl, 4-butoxybenzyl, 3-butoxybenzyl, 2-butoxybenzyl, 2,4-dimethoxybenzyl, 3,4-dimethoxybenzyl, 3,4,5-trimethoxybenzyl, 2-hydroxybenzyl, 3-hydroxybenzyl, 4-hydroxybenzyl, 2,4-dihydroxybenzyl, 3,4-dihydroxybenzyl, 3,4,5-trihydroxybenzyl, 2-hydroxy-4-methoxybenzyl, 3-hydroxy-4-methoxybenzyl, 4-hydroxy-3-methoxybenzyl, 2-methylbenzyl, 3-methylbenzyl, 4-methylbenzyl, 2-ethylbenzyl, 3-ethylbenzyl, 4-ethylbenzyl, 2-propylbenzyl, 3-propylbenzyl, 4-propylbenzyl,

phenylethyl, 4-methoxyphenylethyl, 3-methoxyphenylethyl, 2-methoxyphenylethyl, 4-ethoxyphenylethyl, 3-ethoxyphenylethyl, 2-ethoxyphenylethyl, 4-propoxyphenylethyl, 3-propoxyphenylethyl, 2-propoxyphenylethyl, 4-butoxyphenylethyl, 3-butoxyphenylethyl, 2-butoxyphenylethyl, 2,4-dimethoxyphenylethyl, 3,4-dimethoxyphenylethyl, 3,4,5-trimethoxyphenylethyl, 2-hydroxyphenylethyl, 3-hydroxyphenylethyl, 4-hydroxyphenylethyl, 2,4-dihydroxyphenylethyl, 3,4-dihydroxyphenylethyl, 3,4,5-trihydroxyphenylethyl, 2-hydroxy-4-methoxyphenylethyl, 3-hydroxy-4-methoxyphenylethyl, 4-hydroxy-3-methoxyphenylethyl, 2-methylphenylethyl, 3-methylphenylethyl, 4-methylphenylethyl, 2-ethylphenylethyl, 3-ethylphenylethyl, 4-ethylphenylethyl, 2-propylphenylethyl, 3-propylphenylethyl, 4-propylphenylethyl,

benzenesulfonyl, 2-methoxybenzenesulfonyl, 3-methoxybenzenesulfonyl, 4-methoxybenzenesulfonyl, 4-ethoxybenzenesulfonyl, 3-ethoxybenzenesulfonyl, 2-ethoxybenzenesulfonyl, 4-propoxybenzenesulfonyl, 3-propoxybenzenesulfonyl, 2-propoxybenzenesulfonyl, 4-butoxybenzenesulfonyl, 3-butoxybenzenesulfonyl, 2-butoxybenzenesulfonyl, 2,4-dimethoxybenzenesulfonyl, 3,4-dimethoxybenzenesulfonyl, 3,4,5-trimethoxybenzenesulfonyl, 2-hydroxybenzenesulfonyl, 3-hydroxybenzenesulfonyl, 4-hydroxybenzenesulfonyl, 2,4-dihydroxybenzenesulfonyl, 3,4-dihydroxybenzenesulfonyl, 3,4,5-trihydroxybenzenesulfonyl, 2-hydroxy-4-methoxybenzenesulfonyl, 3-hydroxy-4-methoxybenzenesulfonyl, 4-hydroxy-3-methoxybenzenesulfonyl, 2-methylbenzenesulfonyl, 3-ethylbenzenesulfonyl, 4-methylbenzenesulfonyl, 2-ethylbenzenesulfonyl, 3-ethylbenzenesulfonyl, 4-ethylbenzenesulfonyl, 2-propylbenzenesulfonyl, 3-propylbenzenesulfonyl, 4-propylbenzenesulfonyl,

benzylsulfonyl, 2-methoxybenzylsulfonyl, 3-methoxybenzylsulfonyl, 4-methoxybenzylsulfonyl, 4-ethoxybenzylsulfonyl, 3-ethoxybenzylsulfonyl, 2-ethoxybenzylsulfonyl, 4-propoxybenzylsulfonyl, 3-propoxybenzylsulfonyl, 2-propoxybenzylsulfonyl, 4-butoxybenzylsulfonyl, 3-butoxybenzylsulfonyl, 2-butoxybenzylsulfonyl, 2,4-dimethoxybenzylsulfonyl, 3,4-dimethoxybenzylsulfonyl, 3,4,5-trimethoxybenzylsulfonyl, 2-hydroxybenzylsulfonyl, 3-hydroxybenzylsulfonyl, 4-hydroxybenzylsulfonyl, 2,4-dihydroxybenzylsulfonyl, 3,4-dihydroxybenzylsulfonyl, 3,4,5-trihydroxybenzylsulfonyl, 2-hydroxy-4-methoxybenzylsulfonyl, 3-hydroxy-4-methoxybenzylsulfonyl, 4-hydroxy-3-methoxybenzylsulfonyl, 2-methylbenzylsulfonyl, 3-methylbenzylsulfonyl, 4-methylbenzylsulfonyl, 2-ethylbenzylsulfonyl, 3-ethylbenzylsulfonyl, 4-ethylbenzylsulfonyl, 2-propylbenzylsulfonyl, 3-propylbenzylsulfonyl, 4-propylbenzylsulfonyl,

phenylethylsulfonyl, 4-methoxyphenylethylsulfonyl, 3-methoxyphenylethylsulfonyl, 2-methoxyphenylethylsulfonyl, 4-ethoxyphenylethylsulfonyl, 3-ethoxyphenylethylsulfonyl, 2-ethoxyphenylethylsulfonyl, 4-propoxyphenylethylsulfonyl, 3-propoxyphenylethylsulfonyl, 2-propoxyphenylethylsulfonyl, 4-butoxyphenylethylsulfonyl, 3-butoxyphenylethylsulfonyl, 2-butoxyphenylethylsulfonyl, 2,4-dimethoxyphenylethylsulfonyl, 3,4-dimethoxyphenylethylsulfonyl, 3,4,5-trimethoxyphenylethylsulfonyl, 2-hydroxyphenylethylsulfonyl, 3-hydroxyphenylethylsulfonyl, 4-hydroxyphenylethylsulfonyl, 2,4-dihydroxyphenylethylsulfonyl, 3,4-dihydroxyphenylethylsulfonyl, 3,4,5-trihydroxyphenylethylsulfonyl, 2-hydroxy-4-methoxyphenylethylsulfonyl, 3-hydroxy-4-methoxyphenylethylsulfonyl, 4-hydroxy-3-methoxyphenylethylsulfonyl, 2-methylphenylethylsulfonyl, 3-methylphenylethylsulfonyl, 4-methylphenylethylsulfonyl, 2-ethylphenylethylsulfonyl, 3-ethylphenylethylsulfonyl, 4-ethylphenylethylsulfonyl, 2-propylphenylethylsulfonyl, 3-propylphenylethylsulfonyl, and 4-propylphenylethylsulfonyl groups.

In the general formula (5), n is represents an integer of 0, 1, or 2, as in general formula (4). When n is 0, the compound of the present invention is an *N-*benzoyl-β-alanine derivative corresponding to R₇ through R₁₁. When n is 1, the compound of the present invention is an *N*-phenylacetyl-β-alanine derivative corresponding to R₇ through R₁₁, and when n is 2, the compound of the present invention is an *N*-phenylpropionyl-β-alanine derivative corresponding to R₇ through R₁₁.

Moreover, in general formula (5), R₇ through R₁₁ each independently represent a hydrogen atom, hydroxyl group, C₁₋₄ alkyloxy group, C₁₋₄ alkenyloxy group, C₁₋₃ alkyl group, or C₁₋₃ alkenyl group. When the number of carbon atoms in the alkyloxy or alkenyloxy groups exceeds 4, the effect of the present invention is inferior because solubility in a pharmaceutical preparation is poor and the molecular weight increases at the same time. Any alkyloxy or alkenyloxy group can be used as long as the hydrocarbon segment is a C₁₋₄ segment, and specific examples are methyl, ethyl, *n*-propyl, *iso*-propyl, *n*-butyl, *iso*-butyl, *tert*-butyl, 1-methylpropyl, allyl(2-propenyl), 2-butenyl, 3-butenyl, 1-methyl-2-propenyl, 2-methyl-2-propenyl, cyclopropyl, cyclobutyl, cyclopropylmethyl, cyclopropenyl, cyclopropenylmethyl, and cyclobutenyl groups.

When the number of carbon atoms in the alkyl or alkenyl group exceeds 3, the effect of the present invention will be inferior because solubility in a pharmaceutical preparation is poor and the molecular weight increases at the same time. Any alkyl or alkenyl group can be used as long as the hydrocarbon segment is a C₁₋₃ segment. Specific examples are methyl, ethyl, *n*-propyl, *iso*-propyl, allyl(2-propenyl), cyclopropyl, and cyclopropenyl groups.

In terms of ease of compound synthesis, it is preferred that R₇ through R₁₁ in the general formula (5) each independently be a hydrogen, hydroxyl group, or C₁₋₄ alkyloxy group. It is preferred that each of R₇ through R₁₁ be a hydrogen atom, or that one or more of R₇ through R₁₁ be a C₁₋₄ alkyloxy group because the effect of the present invention will be good. It is preferred that the C₁₋₄ alkyloxy group be a methoxy group because solubility will be good.

Specific examples of groups represented by general formula (5) are benzoyl, p-anisoyl(4-methoxybenzoyl), m-anisoyl(3-methoxybenzoyl), o-anisoyl(2-methoxybenzoyl), 4-ethoxybenzoyl, 3-ethoxybenzoyl, 2-ethoxybenzoyl, 4-propoxybenzoyl, 3-propoxybenzoyl, 2-propoxybenzoyl, 4-butoxybenzoyl, 3-butoxybenzoyl, 2-butoxybenzoyl, 2,4-dimethoxybenzoyl, 3,4-dimethoxybenzoyl, 3,4,5-trimethoxybenzoyl, 2,3,4-trimethoxybenzoyl, 2-hydroxybenzoyl (salicyl), 3-hydroxybenzoyl, 4-hydroxybenzoyl, *N*-2,4-dihydroxybenzoyl, *N*-3,4-dihydroxybenzoyl, *N*-3,4,5-trihydroxybenzoyl (galloyl), 2-hydroxy-4-methoxybenzoyl, 3-hydroxy-4-methoxybenzoyl, 4-hydroxy-3-methoxybenzoyl, 2-methylbenzoyl (*o*-toluyl), 3-methylbenzoyl (m-toluyl), 4-methylbenzoyl (*p*-toluyl), 2-ethylbenzoyl, 3-ethylbenzoyl, 4-ethylbenzoyl, 2-propylbenzoyl, 3-propylbenzoyl, 4-propylbenzoyl,

phenylacetyl, 4-methoxyphenylacetyl, 3-methoxyphenylacetyl, 2-methoxyphenylacetyl, 4-ethoxyphenylacetyl, 3-ethoxyphenylacetyl, 2-ethoxyphenylacetyl, 4-propoxyphenylacetyl, 3-propoxyphenylacetyl, 2-propoxyphenylacetyl, 4-butoxyphenylacetyl, 3-butoxyphenylacetyl, 2-butoxyphenylacetyl, 2,4-dimethoxyphenylacetyl, 3,4-dimethoxyphenylacetyl, 3,4,5-trimethoxyphenylacetyl, 2-hydroxyphenylacetyl, 3-hydroxyphenylacetyl, 4-hydroxyphenylacetyl, N-2,4-dihydroxyphenylacetyl, N-3,4-dihydroxyphenylacetyl, N-3,4,5-trihydroxyphenylacetyl, 2-hydroxy-4-methoxyphenylacetyl, 3-hydroxy-4-methoxyphenylacetyl, 4-hydroxy-3-methoxyphenylacetyl, 2-methylphenylacetyl, 3-methylphenylacetyl, 4-methylphenylacetyl, 2-ethylphenylacetyl, 3-ethylphenylacetyl, 4-ethylphenylacetyl, 2-propylphenylacetyl, 3-propylphenylacetyl, 4-propylphenylacetyl,

phenylpropionyl, 4-methoxyphenylpropionyl, 3-methoxyphenylpropionyl, 2-methoxyphenylpropionyl, 4-ethoxyphenylpropionyl, 3-ethoxyphenylpropionyl, 2-ethoxyphenylpropionyl, 4-propoxyphenylpropionyl, 3-propoxyphenylpropionyl, 2-propoxyphenylpropionyl, 4-butoxyphenylpropionyl, 3-butoxyphenylpropionyl, 2-butoxyphenylpropionyl, 2,4-dimethoxyphenylpropionyl, 3,4-dimethoxyphenylpropionyl, 3,4,5-trimethoxyphenylpropionyl, 2-hydroxyphenylpropionyl, 3-hydroxyphenylpropionyl, 4-hydroxyphenylpropionyl, *N-*2,4-dihydroxyphenylpropionyl, *N*-3,4-dihydroxyphenylpropionyl, *N*-3,4,5-trihydroxyphenylpropionyl, 2-hydroxy-4-methoxyphenylpropionyl, 3-hydroxy-4-methoxyphenylpropionyl, 4-hydroxy-3-methoxyphenylpropionyl, 2-methylphenylpropionyl, 3-methylphenylpropionyl, 4-methylphenylpropionyl, 2-ethylphenylpropionyl, 3-ethylphenylpropionyl, 4-ethylphenylpropionyl, 2-propylphenylpropionyl, 3-propylphenylpropionyl, and 4-propylphenylpropionyl groups.

Combinations of the R₅ and R₆ in the β-alanine derivative represented by general formula (3) according to the present invention may be chosen freely, and the aforementioned limitations do not apply. It is preferred that one of R₅ and R₆ be, e.g., a hydrogen atom or a C₁₋₃ alkyl group because the effect of the present invention will be good. Examples of C₁-₃ alkyl groups are methyl, ethyl, *n*-propyl, and *iso*-propyl groups. Of these, a methyl group is preferred. It is further preferred that one of R₅ and R₆ be a hydrogen atom. R₅ and R₆ cannot both be a hydrogen atom.

When one of the R₅ and R₆ in the β-alanine derivative represented by general formula (3) of the present invention is a C₁₋₇ alkyl or C₁₋₇ alkenyl group, (the other) one is preferably a C₁₋₃ alkyl group, C₁₋₃ alkenyl group, or hydrogen atom because the effect of the present invention will be good. It is more preferable that (the other) one be a hydrogen atom. It is further preferred that one of R₅ and R₆ be a C₁₋₃ alkyl or C₁₋₃ alkenyl group and that (the other) one be a hydrogen atom. It is particularly preferred that one of R₅ and R₆ be a C₁₋₃ alkyl group and that (the other) one be a hydrogen atom.

When one of the R₅ and R₆ in the β-alanine derivative represented by general formula (3) of the present invention is a C₃₋₇ hydrocarbon group having a cyclic segment, (the other) one may be a hydrogen atom, C₁₋₃ alkyl group, C₁₋₃ alkenyl group, C₃₋₇ hydrocarbon group having a cyclic segment, cyclohexyl carbonyl group, cyclopentyl carbonyl group, nicotinoyl group, isonicotinoyl group, picolinoyl group, nipecotinoyl group, isonipecotinoyl group, N-acetyl nipecotinoyl group, N-acetyl isonipecotinoyl group, benzyloxycarbonyl group, or group represented by general formula (4) or (5).

When one of the R₅ and R₆ in the β-alanine derivative represented by general formula (3) of the present invention is a pyridyl group, (the other) one may be a hydrogen atom, C₁₋₇ alkyl group, C₁₋₇ alkenyl group, C₃₋₇ hydrocarbon group having a cyclic segment, cyclohexyl carbonyl group, cyclopentyl carbonyl group, nicotinoyl group, isonicotinoyl group, picolinoyl group, nipecotinoyl group, isonipecotinoyl group, *N*-acetyl nipecotinoyl group, *N*-acetyl isonipecotinoyl group, benzyloxycarbonyl group, or group represented by general formula (4) or (5).

In terms of ease of synthesis, in cases in which one of R₅ and R₆ in the β-alanine derivative represented by formula (3) of the present invention is a cyclohexyl carbonyl group, cyclopentyl carbonyl group, nicotinoyl group, isonicotinoyl group, picolinoyl group, nipecotinoyl group, isonipecotinoyl group, N-acetyl nipecotinoyl group, N-acetyl isonipecotinoyl group, or benzyloxycarbonyl group, it is preferred that (the other) one be a hydrogen atom, pyridyl group, C₁₋₇ alkyl group, C₁₋₇ alkenyl group, C₃₋₇ hydrocarbon group having a cyclic segment, or group represented by general formula (4).

When one of the R₅ and R₆ in the β-alanine derivative represented by general formula (3) according to the present invention is a group represented by general formula (4), (the other) one may be a hydrogen atom, C₁₋₇ alkyl group, C₁₋₇ alkenyl group, C₃₋₇ hydrocarbon group having a cyclic segment, cyclohexyl carbonyl group, cyclopentyl carbonyl group, nicotinoyl group, isonicotinoyl group, picolinoyl group, nipecotinoyl group, isonipecotinoyl group, *N*-acetyl nipecotinoyl group, *N*-acetyl isonipecotinoyl group, benzyloxycarbonyl group, or group represented by general formula (4) or (5). A hydrogen atom, C₁₋₃ alkyl group, or C₁₋₃ alkenyl group is preferred because the solubility of the present invention will be good. A hydrogen atom is more preferable.

Moreover, in terms of ease of synthesis, when one of the R₅ and R₆ in the β-alanine derivative shown in the general formula (3) according to the present invention is a group represented by general formula (5), it is preferred that (the other) one is a hydrogen atom, a C₁₋₇ alkyl group, a C₁₋₇ alkenyl group, a pyridyl group, a C₃₋₇ hydrocarbon group having a cyclic segment, or a group represented by general formula (4).

It is most preferred that the β-alanine derivative represented by general formula (3) according to the present invention be a β-alanine derivative wherein when one of R₅ and R₆ is a group represented by the general formula (5), each of R₇ through R₁₁ is a hydrogen atom, or one or more of R₇ through R₁₁ is a C₁₋₄ alkyloxy group, and the (other) ones are hydrogen atoms.

Specific examples of the β-alanine derivative represented by general formula (3) are *N*-monomethyl-β-alanine (*N*-methyl-β-alanine), *N*-monoethyl-β-alanine, *N-*mono-*n*-propyl-β-alanine, *N*-mono-*iso*-propyl-β-alanine, *N*-monoallyl-β-alanine;

*N*-cyclohexyl-β-alanine, *N*-cyclohexylmethyl-β-alanine, and *N-*methylcyclohexyl-β-alanine, as well as *N*-methylated, *N*-ethylated, *N*-propylated, and *N*-allylated forms of these β-alanine amino groups;

*N*-(2'-pyridyl)-β**-**alanine, *N*-(3'-pyridyl)-β-alanine, and *N*-(4'-pyridyl)-β-alanine, as well as *N*-methylated, *N*-ethylated, *N*-propylated, and *N*-allylated forms of these β-alanine amino groups;

*N*-cyclohexylcarbonyl-β-alanine (*N*-hexahydrobenzoyl-β-alanine), *N-*cyclopentylcarbonyl-β-alanine, *N*-nicotinoyl-β-alanine, *N*-*iso*-nicotinoyl-β-alanine, *N-*picolinoyl-β-alanine, *N*-nipecotinoyl-β-alanine, *N*-*iso*-nipecotinoyl-β-alanine, *N*-(*N*'-acetylnicotinoyl)-β-alanine, *N*-(*N*'-acetyl nipecotinoyl)-β-alanine, *N*-(*N*'-acetyl-*iso*nipecotinoyl)-β-alanine and *N*-benzyloxycarbonyl-β-alanine, as well as *N*-methylated, *N*-ethylated, *N*-propylated, and *N*-allylated forms of these β-alanine amino groups;

*N*-phenyl-β-alanine, *N*-4'-methoxyphenyl-β-alanine, *N*-3'-methoxyphenyl-β-alanine, *N*-2'-methoxyphenyl-β-alanine, *N*-4'-ethoxyphenyl-β-alanine, *N*-3'-ethoxyphenyl-β-alanine, *N*-2'-ethoxyphenyl-β-alanine, *N*-4'-propoxyphenyl-β-alanine, *N*-3'-propoxyphenyl-β-alanine, *N*-2'-propoxyphenyl-β-alanine, *N*-4',butoxyphenyl-β-alanine, *N*-3'-butoxyphenyl-β-alanine, *N*-2'-butoxyphenyl-β-alanine, *N*-2',4'-dimethoxyphenyl-β-alanine, *N*-3',4'-dimethoxyphenyl-β-alanine, *N*-3',4',5'-trimethoxyphenyl-β-alanine, *N*-2'-hydroxyphenyl-β-alanine, *N*-3'-hydroxyphenyl-β-alanine, *N*-4'-hydroxyphenyl-β-alanine, *N*-2',4'-dihydroxyphenyl-β-alanine, *N*-3',4'-dihydroxyphenyl-β-alanine, *N*-3',4',5'-trihydroxyphenyl-β-alanine, *N*-2'-hydroxy-4'-methoxyphenyl-β-alanine, *N*-(2'-hydroxy-4'-methoxyphenyl)-β-alanine, *N*-(3'-hydroxy-4'-methoxyphenyl)-β-alanine, *N*-(4'-hydroxy-3'-methoxyphenyl)-β-alanine, *N*-2'-methylphenyl-β-alanine, *N*-3'-methylphenyl-β-alanine, *N*-4'-methylphenyl-β-alanine, *N*-2'-ethylphenyl-β-alanine, *N*-3'-ethylphenyl-β-alanine, *N*-4'-ethylphenyl-β-alanine, *N*-2'-propylphenyl-β-alanine, *N*-3'-propylphenyl-β-alanine, *N*-4'-propylphenyl-β-alanine,

*N*-benzyl-β-alanine, *N*-4'-methoxybenzyl-β-alanine, *N*-3'-methoxybenzyl-β-alanine, *N*-2'-methoxybenzyl-β-alanine, *N*-4'-ethoxybenzyl-β-alanine, *N*-3'-ethoxybenzyl-β-alanine, *N*-2'-ethoxybenzyl-β-alanine, *N*-4'-propoxybenzyl-β-alanine, *N*-3'-propoxybenzyl-β-alanine, *N*-2'-propoxybenzyl-β-alanine, *N*-4'-butoxybenzyl-β-alanine, *N*-3'-butoxybenzyl-β-alanine, *N*-2'-butoxybenzyl-β-alanine, *N*-2',4'-dimethoxybenzyl-β-alanine, *N*-3',4'-dimethoxybenzyl-β-alanine, *N*-3',4',5'-trimethoxybenzyl-β-alanine, *N*-2'-hydroxybenzyl-β-alanine, *N*-3'-hydroxybenzyl-β-alanine, *N*-4'-hydroxybenzyl-β-alanine, *N*-2',4'-dihydroxybenzyl-β-alanine, *N*-3',4'-dihydroxybenzyl-β-alanine, *N*-3',4',5'-trihydroxybenzyl-β-alanine, *N*-(2'-hydroxy-4'-methoxybenzyl)-β-alanine, *N*-(3'-hydroxy-4'-methoxybenzyl)-β-alanine, *N*-(4'-hydroxy-3'-methoxybenzyl)-β-alanine, *N*-2'-methylbenzyl-β-alanine, *N*-3'-methylbenzyl-β-alanine, *N*-4'-methylbenzyl-β-alanine, *N*-2'-ethylbenzyl-β-alanine, *N-*3'-ethylbenzyl-β-alanine, *N*-4'-ethylbenzyl-β-alanine, *N*-2'-propylbenzyl-β-alanine, *N-*3'-propylbenzyl-β-alanine, *N*-4'-propylbenzyl-β-alanine,

*N*-phenylethyl-β-alanine, *N*-4'-methoxyphenylethyl-β-alanine, *N*-3'-methoxyphenylethyl-β-alanine, *N*-2'-methoxyphenylethyl-β-alanine, *N*-4'-ethoxyphenylethyl-β-alanine, *N*-3'-ethoxyphenylethyl-β-alanine, *N*-2'-ethoxyphenylethyl-β-alanine, *N*-4'-propoxyphenylethyl-β-alanine, *N*-3'-propoxyphenylethyl-β-alanine, *N*-2'-propoxyphenylethyl-β-alanine, *N*-4'-butoxyphenylethyl-β-alanine, *N*-3'-butoxyphenylethyl-β-alanine, *N*-2'-butoxyphenylethyl-β-alanine, *N*-2',4'-dimethoxyphenylethyl-β-alanine, *N*-3',4'-dimethoxyphenylethyl-β-alanine, *N*-3',4',5'-trimethoxyphenylethyl-β-alanine, *N*-2'-hydroxyphenylethyl-β-alanine, *N*-3'-hydroxyphenylethyl-β-alanine, *N*-4'-hydroxyphenylethyl-β-alanine, *N*-2',4'-dihydroxyphenylethyl-β-alanine, *N*-3',4'-dihydroxyphenylethyl-β-alanine, *N*-3',4',5'-trihydroxyphenylethyl-β-alanine, *N*-(2'-hydroxy-4'-methoxyphenylethyl)-β-alanine, *N*-(3'-hydroxy-4'-methoxyphenylethyl)-β-alanine, *N*-(4'-hydroxy-3'-methoxyphenylethyl)-β-alanine, *N*-2'-methylphenylethyl-β-alanine, *N*-3'-methylphenylethyl-β-alanine, *N*-4'-methylphenylethyl-β-alanine, *N*-2'-ethylphenylethyl-β-alanine, *N*-3'-ethylphenylethyl-β-alanine, *N*-4'-ethylphenylethyl-β-alanine, *N*-2'-propylphenylethyl-β-alanine, *N*-3'-propylphenylethyl-β-alanine, *N*-4'-propylphenylethyl-β-alanine,

*N*-benzenesulfonyl-β-alanine, *N*-4'-methoxybenzenesulfonyl-β-alanine, *N*-3'-methoxybenzenesulfonyl-β-alanine, *N*-2'-methoxybenzenesulfonyl-β-alanine, *N-*benzylsulfonyl-β-alanine, *N*-4'-methoxybenzylsulfonyl-β-alanine, *N*-3'-methoxybenzylsulfonyl-β-alanine, *N*-2'-methoxybenzylsulfonyl-β-alanine, *N-*phenylethyl-β-alanine, *N*-4'-methoxyphenylethylsulfonyl-β-alanine, *N*-3'-methoxyphenylethylsulfonyl-β-alanine, and *N*-2'-methoxyphenylethylsulfonyl-β-alanine, as well as *N*-methylated, *N*-ethylated, *N*-propylated, and *N*-altylated forms of these β-alanine amino groups; and

N-benzoyl-β-alanine, *N*-p-anisoyl-β-alanine (*N*-4'-methoxybenzoyl-β-alanine), *N*-m-anisoyl-β-alanine (*N*-3'-methoxybenzoyl-β-alanine), *N*-*o*-anisoyl-β-alanine (*N*-2'-methoxybenzoyl-β-alanine), *N*-4'-ethoxybenzoyl-β-alanine, *N*-3'-ethoxybenzoyl-β-alanine, *N*-2'-ethoxybenzoyl-β-alanine, *N*-4'-propoxybenzoyl-β-alanine, *N*-3'-propoxybenzoyl-β-alanine, *N*-2'-propoxybenzoyl-β-alanine, *N*-4'-butoxybenzoyl-β-alanine, *N*-3'-butoxybenzoyl-β-alanine, *N*-2'-butoxybenzoyl-β-alanine, *N*-2',4'-dimethoxybenzoyl-β-alanine, *N*-3',4'-dimethoxybenzoyl-β-alanine, *N*-3',4',5'-trimethoxybenzoyl-β-alanine, *N*-2'-hydroxybenzoyl-β-alanine (*N*-salicyl-β-alanine), *N-*3'-hydroxybenzoyl-β-alanine, *N*-4'-hydroxybenzoyl-β-alanine, *N*-2',4'-dihydroxybenzoyl-β-alanine,

*N*-3',4'-dihydroxybenzoyl-β-alanine, *N*-3',4',5-trihydroxybenzoyl-β-alanine (*N-*galloyl-β-alanine), *N*-(2'-hydroxy-4'-methoxybenzoyl)-βalanine, *N*-(3'-hydroxy-4'-methoxybenzoyl)-β-alanine, *N*-(4'-hydroxy-3'-methoxybenzoyl)-β-alanine, *N*-2'-methylbenzoyl-β-alanine (*N*-*o*-toluyl-β-alanine), *N*-3'-methylbenzoyl-β-alanine (*N*-*m-*toluyl-β-alanine), *N*-4'-methylbenzoyl-β-alanine (*N*-*p*-toluyl-β-alanine), *N*-2'-ethylbenzoyl-β-alanine, *N*-3'-ethylbenzoyl-β-alanine, *N*-4'-ethylbenzoyl-β-alanine, *N-*2'-propylbenzoyl-β-alanine, *N*-3'-propylbenzoyl-β-alanine, *N*-4'-propylbenzoyl-β-alanine, *N*-phenylacetyl-β-alanine, 4-methoxyphenylacetyl-β-alanine, 3-methoxyphenylacetyl-β-alanine, 2-methoxyphenylacetyl-β-alanine, 4-ethoxyphenylacetyl-β-alanine, 3-ethoxyphenylacetyl-β-alanine, 2-ethoxyphenylacetyl-β-alanine, 2-hydroxyphenylacetyl-β-alanine, 3-hydroxyphenylacetyl-β-alanine, 4-hydroxyphenylacetyl-β-alanine, phenylpropionyl-β-alanine, 4-methoxyphenylpropionyl-β-alanine, 3-methoxyphenylpropionyl-β-alanine, and 2-methoxyphenylpropionyl-β-alanine, as well as *N*-methylated, *N*-ethylated, *N-*propylated, and N-allylated forms of these β-alanine amino groups.

The β-alanine derivative represented by the general formulas (1) through (3) of the present invention can be a salt or a combination of salts. Although the present invention is not limited to these, examples of the salt combinations are alkali metal and alkaline earth metal ions such as sodium, potassium, calcium, zinc, and magnesium; ammonium ions; other amine ions such as methylamine, pyridine, trimethylamine, and triethanol amine; acids such as hydrochloric acid, sulfuric acid, phosphoric acid, hydrobromic acid, and alkylsulfuric acids such as methyl sulfuric acid and *p*-toluenesulfonic acid, as well as acetic acid, lactic acid, maleic acid, fumaric acid, oxalic acid, succinic acid, tartaric acid, and citric acid; and amino acids such as betaine, glycine, serine, and taurine.

When the β-alanine derivative of the present invention is *N*-benzoyl-β-alanine, the wrinkle-improving effect is good, solubility in pharmaceutical preparations is also good, and sensory irritation and other safety issues are not a concern. The compound is very safe and is exceptional in attaining the objects of the present invention.

It is a novel feature that the β-alanine derivatives represented by the general formulas (1), (2), and (3) according to the present invention and salts thereof have a wrinkle-improving effect.

The β-alanine derivative represented by general formula (1) according to the present invention can be easily obtained by, e.g., introducing the corresponding *N-*substitution group to β-alanine or a corresponding analog. In particular, when the β-alanine derivative represented by general formula (1) according to the present invention is 3-(1'-piperidine)-propionic acid, the compound is a conventional material that can be easily synthesized by conventional methods or can be easily obtained as a commercial product from Sigma-Aldrich Co.

The β-alanine derivative represented by general formula (2) according to the present invention can be easily synthesized by, e.g., introducing the corresponding amide group to β-alanine or a corresponding analog. In particular, when the β-alanine derivative represented by general formula (2) according to the present invention is β-alanine amide hydrochloride, the compound is a conventional material that can be easily synthesized by conventional methods or can be easily obtained as a commercial product from Tokyo Chemical Industry Co., Ltd.

The β-alanine derivative represented by general formula (3) comprising a C₁₋₇ alkyl group, C₁₋₇ alkenyl group, or C₃₋₇ hydrocarbon group having a cyclic segment can be easily obtained by introducing the corresponding N-alkyl group, *N*-alkenyl group, or hydrocarbon group having an *N*-cyclic segment to β-alanine or a corresponding analog.

Although it is possible to easily synthesize a β-alanine derivative represented by general formula (3) and having pyridyl groups, when the β-alanine derivative is *N-*(2'-pyridyl)-β-alanine, the compound is a conventional material that in particular can be easily synthesized by conventional methods or can be easily obtained as a commercial product from Wako Pure Chemical Industries, Ltd.

Moreover, the β-alanine derivative represented by general formula (3) and having groups represented by general formula (4) can be easily obtained by methods such as introduction of the corresponding *N*-phenyl, *N*-benzyl, *N-*phenylethyl, *N*-benzenesulfonyl, *N*-benzylsulfonyl, or *N*-phenylethylsulfonyl group to β-alanine or a corresponding analog; removal of protector groups after introduction of the protected corresponding phenyl, benzyl, phenylethyl, *N*-benzenesulfonyl, *N-*benzylsulfonyl, or *N*-phenylethylsulfonyl; selective alkylation of phenolic hydroxyl groups after introduction of the corresponding phenyl, benzyl, phenylethyl, *N-*benzenesulfonyl, *N*-benzylsulfonyl, or N-phenylethylsulfonyl group; or introduction of alkyl groups to the aromatic ring by Friedel-Crafts reaction or the like after introduction of the corresponding phenyl, benzyl, phenylethyl, *N*-benzenesulfonyl, *N-*benzylsulfonyl, or N-phenylethylsulfonyl group.

Moreover, the β-alanine derivative represented by general formula (3) and having groups represented by general formula (5) can be easily obtained by methods such as introduction of the corresponding *N*-benzoyl, *N*-phenylacetyl, or *N-*phenylpropyloyl group to β-alanine or a corresponding analog; removal of protector groups after introduction of the protected corresponding benzoyl, phenylacetyl, or phenylpropyloyl group; selective alkylation of phenolic hydroxyl groups after introduction of the corresponding benzoyl, phenylacetyl, or phenylpropyloyl group; or introduction of alkyl or alkenyl groups to the aromatic ring by Friedel-Crafts reaction or the like after introduction of the corresponding benzoyl, phenylacetyl, or phenylpropyloyl group.

In particular, when the β-atanine derivative shown in the general formula (3) that has the groups shown in general formula (5) according to the present invention is *N*-benzoyl-β-alanine, the compound is a conventional material that can be easily synthesized by conventional methods or can be easily obtained as a commercial product from Tokyo Chemical Industry Co., Ltd.

The following are typical examples of synthesizing the β-alanine derivative according to the present invention, but the present invention is not limited to these examples.

(Synthesis Examples)

### 1) N-o-anisoyl-β-alanine (N-2'-methoxybenzoyl-β-alanine)

Ten grams of β-alanine and 9 g of sodium hydroxide were dissolved in 100 mL of purified water, and 17.4 g of o-anisoyl chloride was added dropwise under freezing conditions. After stirring for six hours, hydrochloric acid was added and the pH was adjusted to 2 or less. After extraction with 500 mL of ethyl acetate, the product was desiccated over magnesium sulfate, filtered, and concentrated under reduced pressure. The resulting residue was recrystallized with aqueous ethanol, and 17.0 g of the desired product was obtained (yield of 75%).

### 2) N-m-anisoyl-β-alanine (N-3'-methoxybenzoyl-β-alanine)

Ten grams of β-alanine and 9 g of sodium hydroxide were dissolved in 100 mL of purified water, and 17.4 g of *m*-anisoyl chloride was added dropwise under freezing conditions. After stirring for five hours, hydrochloric acid was added and the pH was adjusted to 2 or less. After extraction with 500 mL of ethyl acetate, the product was desiccated over magnesium sulfate, filtered, and concentrated under reduced pressure. The resulting residue was recrystallized with aqueous ethanol, and 18.5 g of the desired product was obtained (yield of 81 %).

### 3) N-p-anisoyl-β-alanine (N-4'-methoxybenzoyl-β-alanine)

Ten grams of β-alanine and 9 g of sodium hydroxide were dissolved in 100 mL of purified water, and 17.4 g of *p*-anisoyl chloride was added dropwise under freezing conditions. After stirring for five hours, hydrochloric acid was added and the pH was adjusted to 2 or less. After extraction with 500 mL of ethyl acetate, the product was desiccated over magnesium sulfate, filtered, and concentrated under reduced pressure. The resulting residue was recrystallized with aqueous ethanol, and 19.1 g of the desired product was obtained (yield of 84%).

### 4) N-cyclohexylcarbonyl-β-alanine (N-hexahydrobenzoyl-β-alanine)

Ten grams of β-alanine and 9 g of sodium hydroxide were dissolved in 100 mL of purified water, and 14.9 g of cyclohexanecarbonyl chloride was added dropwise under freezing conditions. After stirring for five hours, hydrochloric acid was added and the pH was adjusted to 2 or less. After extraction with 500 mL of ethyl acetate, the product was desiccated over magnesium sulfate, filtered, and concentrated under reduced pressure. The resulting residue was recrystallized with aqueous ethanol, and 17.2 g of the desired product was obtained (yield of 85%).

### 5) N-nicotinoyl-β-alanine

Ten grams of β-alanine and 9 g of sodium hydroxide were dissolved in 100 mL of purified water, and 18.15 g of nicotinoyl chloride hydrochloride was added dropwise under freezing conditions. After stirring for five hours, the product was filtered, the resulting crude product was recrystallized with aqueous ethanol, and 19.1 g of the desired product was obtained (yield of 84%).

### 6) N-benzyl-β-alanine

Ten grams of β-alanine ethyl ester hydrochloride and 9 g of benzaldehyde were added to 100 mL of toluene and heated and refluxed using a Dean-Stark trap. After heating and stirring for three hours, the product was cooled in air, and the reaction system was concentrated under reduced pressure. 200 mL of ethanol were added to the resulting residue, and 5 g of sodium borohydride was added while stirring under freezing conditions. After stirring over a period of one night, hydrochloric acid was added and the pH was brought to 2 or less. After filtering, the product was concentrated under reduced pressure, the resulting residue was dissolved in 200 mL THF-water, and 2 g of sodium hydroxide were added. After stirring for six hours at room temperature, the product was neutralized with Amberlite IR120B[H⁺] and filtered, and the filtrate was concentrated under reduced pressure. The resulting residue was recrystallized, and 8.2 g of the desired product was obtained (yield of 70%).

### 7) N-methyl-β-alanine

After methylamine was blown into 10 mL of pyridine, 1 g of β-bromopropionic acid was added, and the reactants were reacted overnight in a sealed vessel at 90°C. The reaction system was concentrated under reduced pressure. 20 mL of ethanol was added to the resulting residue, the product was neutralized with Amberlite IR120B[H⁺] and then filtered, and the filtrate was concentrated under reduced pressure. The resulting residue was recrystallized, and 890 mg of the desired product was obtained (yield of 80%).

### 8) N-cyclohexyl-β-alanine

22 g of cyclohexylamine was added to 40 mL of ethanol, 10 g of ethyl β-bromopropionate were added, and the mixture was heated and refluxed for two hours. The reaction system was concentrated under reduced pressure, the resulting residue was extracted with 200 mL of ethyl acetate, the organic layer was desiccated over anhydrous magnesium sulfate, and the product was then filtered and concentrated. The residue was purified by distillation under reduced pressure, and 6.3 g of *N*-cyclohexylmethyl β-alanine ethyl ester (yield of 57%) was obtained. 1.26 g of sodium hydroxide was dissolved in 200 mL of water, 6.3 g of *N*-cyclohexyl-β-alanine ethyl ester was added, and THF was added for dissolution. After stirring for one hour, the product was neutralized using Dowex 50WX4[H⁺] and filtered, and the filtrate was concentrated under reduced pressure. The resulting residue was recrystallized, and 3.2 g of the desired product was obtained (yield of 64%).

### 9) N-cyclohexyl-β-alanine

22 g of cyclohexyl methylamine was added to 40 mL of ethanol, 10 g of ethyl β-bromopropionate was added, and the mixture was heated and refluxed for two hours. The reaction system was concentrated under reduced pressure, the resulting residue was extracted with 200 mL of ethyl acetate, the organic layer was desiccated over anhydrous magnesium sulfate, and the product was then filtered and concentrated. The residue was purified by distillation under reduced pressure, and 9.7 g of *N*-cyclohexylmethyl-β-alanine ethyl ester (yield of 82%) was obtained. 1.81 g of sodium hydroxide was dissolved in 200 mL of water, 9.7 g of N-cyclohexylmethyl-β-alanine ethyl ester was added, and THF was added for dissolution. After stirring for one hour, the product was neutralized using Dowex 50WX4[H⁺] and filtered, and the filtrate was concentrated under reduced pressure. The resulting residue was recrystallized, and 6.7 g of the desired product was obtained (yield of 80%).

### 10) N-cyclohexyl-N-methyl-β-alanine

22 g of *N*-methylcyclohexylamine was added to 40 mL of ethanol, 10 g of ethyl β-bromopropionate was added, and the mixture was heated and refluxed for two hours. The reaction system was concentrated under reduced pressure, the resulting residue was extracted with 200 mL of ethyl acetate, the organic layer was desiccated over anhydrous magnesium sulfate, and the product was then filtered and concentrated. The residue was purified by distillation under reduced pressure, and 9.4 g of *N*-cyclohexyl-*N*-methyl β-alanine ethyl ester (yield of 80%) ways obtained. 1.75 g of sodium hydroxide was dissolved in 200 mL of water, 9.4 g of *N*-cyclohexyl-N-methyl- β-alanine ethyl ester was obtained, and THF was added for dissolution. After stirring for one hour, the product was neutralized using Dowex 50WX4[H⁺] and filtered, and the filtrate was concentrated under reduced pressure. The resulting residue was recrystallized, and 6.7 g of the desired product was obtained (yield of 82%).

### 11) N-benzenesulfonyl-β-alanine

Ten grams of β-alanine and 9 g of sodium hydroxide were dissolved in 100 mL of purified water, 17.4 g of sulfonyl chloride was added dropwise under freezing conditions, and then the product was stirred for six hours. After washing with 200 mL of ethyl acetate; hydrochloric acid was added to bring the mixture to approximate neutrality; the mixture was extracted using 500 mL of ethyl acetate; the product was desiccated over magnesium sulfate, filtered, and then concentrated under reduced pressure; the resulting residue was recrystallized using aqueous ethanol; and 17.0 g of the desired product was obtained (yield of 75%).

A β-alanine derivative or a salt thereof as represented by the general formula (1), (2), or (3) according to the present invention has an excellent wrinkle-improving effect and thereby improves wrinkles when the compound is applied to the skin. Consequently, a wrinkle-improving agent is prepared that includes as active components one, two, or more compounds selected from the group consisting of β-alanine derivatives represented by the general formula (1), (2), or (3) according to the present invention, and salts thereof (one, two, or more of compounds selected from the group consisting of β-alanine derivatives represented by the general formula (1), (2), or (3) according to the present invention, and salts thereof are also referred to as "β-alanine derivatives" hereafter).

This wrinkle-improving agent is preferably used in the format of an agent for external use on the skin. The wrinkle-improving agent improves wrinkles when used on, e.g., the face or body.

This wrinkle-improving agent is a novel and useful application based on the discovery of the above-mentioned novel function of the β-alanine derivative according to the present invention.

The wrinkle-improving agent according to the present invention is very safe; therefore, it has a very wide application range and can be used in a variety of fields. Preferred examples of such fields include pharmaceutical products, food products, and cosmetics containing quasidrugs.

The β-alanine derivative of the present invention is contained in the wrinkle-improving agent in an amount that is effective for realizing the relevant function. This content is preferably 0.001 to 20.0 mass%, particularly 0.01 to 10.0 mass%, and particularly preferably 0.2 to 5.0 mass%, in relation to the total amount of composition. When a mixture of the β-alanine derivatives or salts thereof is used, the maximum total content is preferably 20.0 mass% or less, even more preferably 10.0 mass% or less, and particularly preferably 5.0 mass% or less.

Components normally used for external agents for skin such as cosmetics and pharmaceutical preparations are optionally added as needed to the wrinkle-improving agent according to the present invention in addition to the necessary constituents described above. Examples of these components are powders, pigments, liquid oils, solid oils, waxes, hydrocarbon oils, higher fatty acids, higher alcohols, synthetic ester oils, silicones, other oils, anionic surfactants, cationic surfactants, ampholytic surfactants, lipophilic non-ionic surfactants, hydrophilic non-ionic surfactants, other surfactants, humectants, natural water-soluble macromolecules, semisynthetic water-soluble macromolecules, synthetic water-soluble polymers, thickeners, coating agents, UV absorbers, sequestrants, lower alcohols, polyalcohols, sugars, amino acids, organic amines, synthetic resin emulsions, pH regulators, skin nutrients, various other medicinally effective components, vitamins, antioxidants, antioxidant auxiliaries, antiseptics, neutralizers, perfumes, and water. Specific examples of components that can be mixed in will be given below, but these components are not given by way of limitation. The wrinkle-improving agent of the present invention can be manufactured by standard methods in which any one, two, or more types of these components are mixed together with the necessary components described above.

The wrinkle-improving agent of the present invention can be widely used in the form of a pharmaceutical product, a quasi-drug (an ointment or the like), or a cosmetic (fundamental cosmetic such as a face wash, milky lotion, cream, gel, essence (clarifier), pack, or mask; makeup such as foundation or lipstick; body cosmetic; and the like). The composition of the wrinkle-improving agent of the present invention is not limited to these forms.

The dosage form can be a variety of dosage forms, including aqueous solutions, microemulsions, emulsions, oils, gels, ointments, aerosols, water-oil two-layer forms, water-oil-powder three-layer forms, and the like.

### [Examples]

Specifics of the present invention will now be described using the following examples. Unless otherwise stated, the amounts added are by mass%.

### [Example 1] Tests of wrinkle improvement on hairless mice

Wrinkles were formed on Hr-1 (Skh-1) hairless mice (age 6 to 10 weeks) according to a partly modified version of the method of Schwartz et al. (see Haratake A. et al. J. Invest. Dermatol., 108, 769-775, 1997), in which the mice were repeatedly irradiated with UVB (see Naganuma M. et al. J. Dermatol. Sci., 25, 29-35, 2001; Schwartz E. J. Invest. Dermatol. 91, 158-161, 1988). Specifically, the backs [of the mice] were irradiated with UVB (light source: Toshiba FL-20 SE fluorescent lamp, made by Toshiba Electric) three times a week for ten weeks. The amount of irradiation after the treatment commenced was 36 mJ/cm²/irradiation. The amount was gradually increased from the second week onwards, and during the tenth week the amount was 216 mJ/cm²/irradiation. The total irradiation amount was 4.6 J/cm². Values for the amount of ultraviolet light were measured used a UV radiometer (UVR-305/365D(II), [made by] Topcon). [After] irradiation with ultraviolet light was completed, photographs were taken of the backs of the mice, and the degree of wrinkle generation was scored according to the wrinkle evaluation standards given below using a partially modified version of the method of Bissett et al. (see Bissett, D.L., et al. Photochemistry and Photobiology, 46, 367-378, 1987). Scores of 0, 2, 4, 6, and 8 were used as standard values, and 1, 3, 5, and 7 were used as intermediate scores. Only mice having a wrinkle score of 7 or more were used in the wrinkle improvement test below. Wrinkle scoring was performed by three measurers working separately, and the score was decided by consensus.

### [Evaluation Standards]

0: No wrinkles seen.
1: Wrinkles shallower, shorter, or smaller in number than those of score 2.
2: Shallow wrinkles seen.
3: Wrinkles deeper or longer than those of score 2, and shallower, shorter, or smaller in number than those of score 4.
4: Shallow wrinkles seen over the entire surface.
5: Wrinkles deeper or longer than those of score 4, and shallower or shorter than those of score 6.
6: Long, deep wrinkles seen.
7: Increased amounts of wrinkles deeper and longer than those of score 6 and shallower or shorter than those of score 8.
8: Long, deep wrinkles seen over the entire surface.

Hairless mice having a score of 7 or more as described above were put into two groups of five mice each and divided so that the scores of both groups were identical. Solvent (50% ethanol) or 3% sample solution (N-benzoyl-β-alanine or the like) (prepared using 50% ethanol as a solvent) were used as test samples, and 100 µL of the test samples was applied to the entire surface of the skin on the backs of the mice in each group once a day, five times a week, continuously for five weeks. Once the [period of] application was completed, photographs were taken of the backs of the mice. The degree of wrinkling was scored according to the aforedescribed wrinkle evaluation standards by three measurers working separately, with the group name of the animal covered, and the score was decided by consensus.

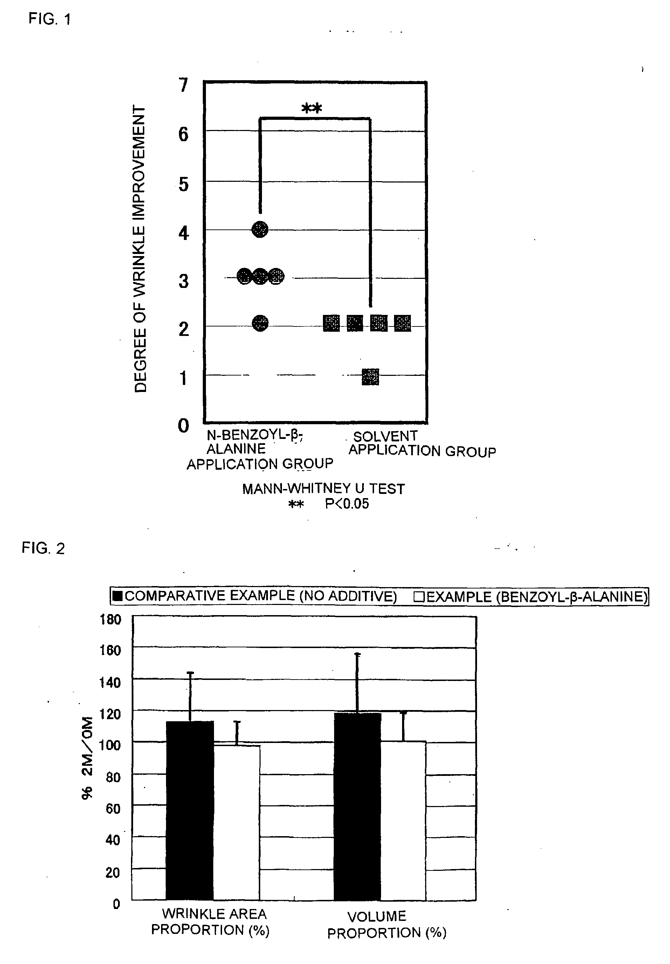

The degree of wrinkle improvement of the mice was determined in the following manner. Calculation was performed using the equation "degree of wrinkle improvement" = "score before sample application" - "score after five weeks of sample application." The Mann-Whitney U test was employed on the scores between the two groups. The degrees of wrinkle improvement for N-benzoyl-β-alanine obtained according to the equation above are shown in FIG. 1.

As is clear from FIG. 1, the reduction in wrinkles dramatically accelerated in the group to which the N-benzoyl-β-alanine solution of the present invention was applied, in contrast to the group to which solvent (50% ethanol) was applied. This result was confirmed to be statistically significant. The above results confirm that the N-benzoyl-β-alanine solution is effective in improving wrinkles formed in skin by ultraviolet light.

### [Example 2] Tests of improvement in wrinkles that form in the corners of the eyes

Face lotions (an example and a comparative example) having the compositions given below were applied three times a day using the half-face method (blind tests) to the faces of healthy male panelists having a large number of wrinkles.

| Face lotion (Example) | |
|---|---|
| Component | Amount added (mass%) |
| Benzoyl-β-alanine | 2.5 |
| Cosmetic alcohol | 15.0 |
| Purified water | Balance |

| Face lotion (Comparative Example) | |
|---|---|
| Component | Amount added (mass%) |
| Cosmetic alcohol | 15.0 |
| Purified water | Balance |

Replicas of the eye corner portions to which lotion was applied were acquired using a SILFLO [apparatus] (Flexico Development Ltd.). The proportion of wrinkle area and the proportion of wrinkle volume were determined for before the application of the face lotion (0 M), after one month of application (1 M), and after two months of application (2 M). Using this data, the ratios of wrinkle area proportion after one and two months of application relative to 100% wrinkle area proportion before application of the face lotion, as well as the ratios of wrinkle volume proportion after one and two months of application relative to 100% wrinkle volume proportion for before application of the face lotion were determined. Both the wrinkle area proportion and the wrinkle volume proportion were determined by analyzing the replicas using a wrinkle-measuring apparatus employing a laser-cutting method (see Japanese Laid-Open Patent Application No. 7-113623).

FIG. 2 shows the average value of the ratio (%) of the wrinkle area after two months of application relative to the wrinkle area before application of the face lotion (100%), and the average value of the ratio (%) of the wrinkle volume after two months of application relative to the wrinkle volume before application of the face lotion (100%). The results were that increases in wrinkle area and wrinkle volume were minimized for the face lotion (Example) (indicated by "Example (benzoyl-β-alanine" in FIG. 2) in comparison to the face lotion (Comparative Example) (indicated by "Comparative Example (No Additive)" in FIG. 2), in which wrinkle area and wrinkle volume increased.

The results of a survey concerning "Impressions of the Effectiveness of Wrinkle improvement" sent to 23 test subjects are shown in Table 1. The results show that more than 60% felt that there was an effect of wrinkle improvement.

**[Table 1]**

| | |
|---|---|
| Persons reporting an effect | 65.2% |
| Persons reporting no effect | 34.8% |

The results above confirm that benzoyl-β-alanine had a wrinkle-improving effect.

Examples of pharmaceutical preparations of the wrinkle-improving agent according to the present invention are shown below. Each of these wrinkle-improving agents had an excellent wrinkle-improving effect.

### Pharmaceutical Preparation 1; Wrinkle Improving Cream

| Component | Amount added (mass%) |
|---|---|
| Stearic acid | 5.0 |
| Stearyl alcohol | 4.0 |
| Isopropyl myristate | 18.0 |
| Glycerin monostearic acid ester | 3.0 |
| Propylene glycol | 10.0 |
| *N*-benzoyl-β-alanine | 20.0 |
| Potassium hydroxide | 0.2 |
| Sodium bisulfite | 0.01 |
| Preservative | As needed |
| Fragrance | As needed |
| Deionized water | Balance |

### (Production Method)

Propylene glycol and potassium hydroxide were added to deionized water and dissolved, heated, and maintained at 70°C (aqueous phase). The other components were mixed, heated, melted, and maintained at 70°C (oil phase). The oil phase was gradually added to the aqueous phase. Once everything had been added, the temperature was maintained for a short time and a reaction was made to occur. [The mixture] was then uniformly emulsified using a homomixer, cooled to 30°C while being thoroughly stirred, and [the product] was manufactured.

### Pharmaceutical Preparations 2 through 19; Wrinkle Improving Cream

The wrinkle-improving creams of pharmaceutical preparations 2 through 19 were prepared as in pharmaceutical preparation 1 by mixing the following components added in place of *N*-benzoyl-β-alanine of the components of Pharmaceutical Preparation 1: *N*-(4'-methoxybenzoyl)-β-alanine (Pharmaceutical Preparation 2), *N*-(3'-methoxybenzoyl)-β-alanine (Pharmaceutical Preparation 3), *N*-(2'-methoxybenzoyl)-β-alanine (Pharmaceutical Preparation 4), 3-(1'-piperidine)-propionic acid (Pharmaceutical Preparation 5), *N*-benzyl-β-alanine (Pharmaceutical Preparation 6), *N*-benzenesulfonyl-β-alanine (Pharmaceutical Preparation 7), *N-*cyclohexyl-β-alanine (Pharmaceutical Preparation 8), *N*-cyclohexylmethyl-β-alanine (Pharmaceutical Preparation 9), *N*-cyclohexyl-*N*-methyl-β-alanine (Pharmaceutical Preparation 10), *N*-(2'-pyridyl)-β-alanine (Pharmaceutical Preparation 11), *N-*nicotinoyl-β-alanine (Pharmaceutical Preparation 12), *N*-benzyloxycarbonyl-β-alanine (Pharmaceutical Preparation 13), β-alanine amide hydrochloride (Pharmaceutical Preparation 14), *N*-3',4',5'-trimethoxybenzoyl-β-alanine (Pharmaceutical Preparation 15), *N*-phenylacetyl-β-alanine (Pharmaceutical Preparation 16), *N-*cyclohexylcarbonyl-β-alanine (Pharmaceutical Preparation 17), 10.0 mass% of *N-*benzoyl-β-alanine, 5.0 mass% of *N*-cyclohexyl-β-alanine, and 5.0 mass% of 3-(1'-piperidine)propionate (Pharmaceutical Preparation 18), and *N*-monomethyl-β-alanine (Pharmaceutical Preparation 19).

### Pharmaceutical Preparation 20; Wrinkle Improving Cream

| Component | Amount added (mass%) |
|---|---|
| Stearic acid | 6.0 |
| Sorbitan monostearic acid ester | 2.0 |
| Polyoxyethylene (20 mol) sorbitan | 1.5 |
| monostearic acid ester | |
| Propylene glycol | 10.0 |
| *N*-benzoyl-β-alanine | 7.0 |
| Glycerin trioctanoate | 10.0 |
| Squalene | 5.0 |
| Sodium bisulfite | 0.01 |
| Ethylparaben | 0.3 |
| Fragrance | As needed |
| Deionized water | Balance |

### (Production Method)

Propylene glycol was added to deionized water and dissolved, heated, and maintained at 70°C (aqueous phase). The other components were mixed, heated, melted, and maintained at 70°C (oil phase). The oil phase was added to the aqueous phase, pre-emulsified, uniformly emulsified using a homomixer, cooled to 30°C while being thoroughly stirred, and [the product] was manufactured.

### Pharmaceutical Preparations 21 through 37; Wrinkle Improving Cream

The wrinkle-improving creams of pharmaceutical preparations 21 through 37 were prepared as in pharmaceutical preparation 20 by mixing the following components added in place of *N*-benzoyl-β-alanine of the components of Pharmaceutical Preparation 20: *N*-(4'-methoxybenzoyl)-β-alanine (Pharmaceutical Preparation 21), *N*-(3'-methoxybenzoyl)-β-alanine (Pharmaceutical Preparation 22), *N*-(2'-methoxybenzoyl)-β-alanine (Pharmaceutical Preparation 23), 3-(1'-piperidine)-propionic acid (Pharmaceutical Preparation 24), *N*-benzyl-β-alanine (Pharmaceutical Preparation 25), *N*-benzenesulfonyl-β-alanine (Pharmaceutical Preparation 26), *N*-cyclohexyl-β-alanine (Pharmaceutical Preparation 27), *N-*cyclohexylmethyl-β-alanine (Pharmaceutical Preparation 28), *N*-cyclohexyl-*N*-methyl-β-alanine (Pharmaceutical Preparation 29), *N*-(2'-pyridyl)-β-alanine (Pharmaceutical Preparation 30), *N*-nicotinoyl-β-alanine (Pharmaceutical Preparation 31), *N-*benzyloxycarbonyl-β-alanine (Pharmaceutical Preparation 32), β-alanine amide hydrochloride (Pharmaceutical Preparation 33), *N*-3',4',5'-trimethoxybenzoyl-β-alanine (Pharmaceutical Preparation 34), *N*-phenylacetyl-β-alanine (Pharmaceutical Preparation 35), *N-*cyclohexylcarbonyl-β-alanine (Pharmaceutical Preparation 36), and *N*-monomethyl-β-alanine (Pharmaceutical Preparation 37).

### Pharmaceutical Preparation 38; Wrinkle Improving Cream

| Component | Amount added (mass%) |
|---|---|
| Stearyl alcohol | 7.0 |
| Stearic acid | 2.0 |
| Hydrogenated lanolin | 2.0 |
| Squalane | 5.0 |
| 2-Octyldodecyl alcohol | 6.0 |
| Polyoxyethylene (25 mol) cetyl alcohol ester | 3.0 |
| Glycerin monostearic acid ester | 2.0 |
| Propylene glycol | 5.0 |
| *N*-benzoyl-β-alanine | 0.001 |
| Fragrance | As needed |
| Sodium bisulfite | 0.03 |
| Ethylparaben | 0.3 |
| Deionized water | Balance |

### (Production Method)

Propylene glycol was added to deionized water and dissolved, heated, and maintained at 70°C (aqueous phase). The other components were mixed, heated, melted, and maintained at 70°C (oil phase). The oil phase was added to the aqueous phase, pre-emulsified, uniformly emulsified using a homomixer, cooled to 30°C while being thoroughly stirred, and [the product] was manufactured.

### Pharmaceutical Preparations 39 through 55; Wrinkle Improving Cream

The wrinkle-improving creams of pharmaceutical preparations 39 through 55 were prepared as in pharmaceutical preparation 38 by mixing the following components added in place of *N*-benzoyl-β-alanine of the components of Pharmaceutical Preparation 38: *N*-(4'-methoxybenzoyl)-β-alanine (Pharmaceutical Preparation 39), *N*-(3'-methoxybenzoyl)-β-alanine (Pharmaceutical Preparation 40), *N*-(2'-methoxybenzoyl)-β-alanine (Pharmaceutical Preparation 41), 3-(1'-piperidine)-propionic acid (Pharmaceutical Preparation 42), *N*-benzyl-β-alanine (Pharmaceutical Preparation 43), *N*-benzenesulfonyl-β-alanine (Pharmaceutical Preparation 44), *N*-cyclohexyl-β-alanine (Pharmaceutical Preparation 45), *N-*cyclohexylmethyl-β-alanine (Pharmaceutical Preparation 46), *N*-cyclohexyl-*N*-methyl-β-alanine (Pharmaceutical Preparation 47), *N*-(2'-pyridyl)-β-alanine (Pharmaceutical Preparation 48), *N*-nicotinoyl-β-alanine (Pharmaceutical Preparation 49), *N-*benzyloxycarbonyl-β-alanine (Pharmaceutical Preparation 50), β-alanine amide hydrochloride (Pharmaceutical Preparation 51), *N*-3',4',5'-trimethoxybenzoyl-β-alanine (Pharmaceutical Preparation 52), *N*-phenylacetyl-β-alanine (Pharmaceutical Preparation 53), *N*-cyclohexylcarbonyl-β-alanine (Pharmaceutical Preparation 54), and *N*-monomethyl-β-alanine (Pharmaceutical Preparation 55).

### Pharmaceutical Preparation 56; Wrinkle-Improving Milky Lotion

| Component | Amount added (mass%) |
|---|---|
| Stearic acid | 2.5 |
| Cetyl alcohol | 1.5 |
| Vaseline | 5.0 |
| Liquid paraffin | 10.0 |
| Polyoxyethylene (10) monooleic acid ester | 2.0 |
| Polyethylene glycol 1500 | 3.0 |
| Triethanolamine | 1.0 |
| *N*-benzoyl-β-alanine | 10.0 |
| Sodium bisulfite | 0.01 |
| Ethylparaben | 0.3 |
| Carboxyvinyl polymer | 0.05 |
| Fragrance | As needed |
| Deionized water | Balance |

### (Production method)

Carboxyvinyl polymer was dissolved in a small amount of deionized water (phase A). Polyethylene glycol 1500 and triethanol amine were added and heated, dissolved into the remaining deionized water, and maintained at 70°C (aqueous phase). The other components were mixed, heated, melted, and maintained at 70°C (oil phase). The oil phase was added to the aqueous phase and pre-emulsified, phase A was added and uniformly emulsified with a homomixer, and the product was cooled to 30°C while being thoroughly agitated.

### Pharmaceutical Preparations 57 through 73; Wrinkle Improving Milky Lotion

The wrinkle-improving milky lotions of pharmaceutical preparations 57 through 73 were prepared as in pharmaceutical preparation 56 by mixing the following components added in place of *N*-benzoyl-β-alanine of the components of Pharmaceutical Preparation 56: *N*-(4'-methoxybenzoyl)-β-alanine (Pharmaceutical Preparation 57), *N*-(3'-methoxybenzoyl)-β-alanine (Pharmaceutical Preparation 58), *N*-(2'-methoxybenzoyl)-β-alanine (Pharmaceutical Preparation 59), 3-(1'-piperidine)-propionic acid (Pharmaceutical Preparation 60), *N*-benzyl-β-alanine (Pharmaceutical Preparation 61), *N*-benzenesulfonyl-β-alanine (Pharmaceutical Preparation 62), *N*-cyclohexyl-β-alanine (Pharmaceutical Preparation 63), *N-*cyclohexylmethyl-β-alanine (Pharmaceutical Preparation 64), *N*-cyclohexyl-*N*-methyl-β-alanine (Pharmaceutical Preparation 65), *N*-(2'-pyridyl)-β-alanine (Pharmaceutical Preparation 66), *N*-nicotinoyl-β-alanine (Pharmaceutical Preparation 67), N-benzyloxycarbonyl-β-alanine (Pharmaceutical Preparation 68), β-alanine amide hydrochloride (Pharmaceutical Preparation 69), *N*-3',4',5'-trimethoxybenzoyl-β-alanine (Pharmaceutical Preparation 70), *N*-phenylacetyl-β-alanine (Pharmaceutical Preparation 71), *N*-cyclohexylcarbonyl-β-alanine (Pharmaceutical Preparation 72), and *N*-monomethyl-β-alanine (Pharmaceutical Preparation 73).

### Pharmaceutical Preparation 74; Wrinkle-Improving Milky Lotion

| Component | Amount added (mass%) |
|---|---|
| (Oil phase) | |
| Stearyl alcohol | 1.5 |
| Squalene | 2.0 |
| Vaseline | 2.5 |
| Deodorized liquid lanolin | 1.5 |
| Evening primrose oil | 2.0 |
| Isopropyl myristate | 5.0 |
| Glycerin monooleate | 2.0 |
| Polyoxyethylene (60 mol) hydrogenated | 2.0 |
| castor oil | |
| Tocopherol acetate | 0.05 |
| Ethylparaben | 0.2 |
| Butylparaben | 0.1 |
| Ethylglycine | 1.0 |
| *N*-benzoyl-β-alanine | 1.0 |
| Fragrance | As needed |

| (Aqueous phase) | |
|---|---|
| Sodium bisulfite | 0.01 |
| Glycerin | 5.0 |
| Sodium hyaluronate | 0.01 |
| Carboxyvinyl polymer | 0.2 |
| Potassium hydroxide | 0.2 |
| Purified water | Balance |

### (Production method)

The oil phase was dissolved at 70°C. The aqueous phase was dissolved at 70°C, the oil phase was mixed with the aqueous phase, and emulsification was performed using an emulsifier. The product was then cooled to 30°C using a heat exchanger.

### Pharmaceutical Preparations 75 through 91; Wrinkle Improving Milky Lotion

The wrinkle-improving milky lotions of pharmaceutical preparations 75 through 91 were prepared as in pharmaceutical preparation 74 by mixing the following components added in place of *N*-benzoyl-β-alanine of the components of Pharmaceutical Preparation 74: *N*-(4'-methoxybenzoyl)-β-alanine (Pharmaceutical Preparation 75), *N*-(3'-methoxybenzoyl)-β-alanine (Pharmaceutical Preparation 76), *N*-(2'-methoxybenzoyl)-β-alanine (Pharmaceutical Preparation 77), 3-(1'-piperidine)-propionic acid (Pharmaceutical Preparation 78), *N*-benzyl-β-alanine (Pharmaceutical Preparation 79), *N*-benzenesulfonyl-β-alanine (Pharmaceutical Preparation 80), *N*-cyclohexyl-β-alanine (Pharmaceutical Preparation 81), *N-*cyclohexylmethyl-β-alanine (Pharmaceutical Preparation 82), *N*-cyclohexyl-*N*-methyl-β-alanine (Pharmaceutical Preparation 83), *N*-(2'-pyridyl)-β-alanine (Pharmaceutical Preparation 84), *N*-nicotinoyl-β-alanine (Pharmaceutical Preparation 85), *N-*benzyloxycarbonyl-β-alanine (Pharmaceutical Preparation 86), β-alanine amide hydrochloride (Pharmaceutical Preparation 87), *N*-3',4',5'-trimethoxybenzoyl-β-alanine (Pharmaceutical Preparation 88), *N*-phenylacetyl-β-alanine (Pharmaceutical Preparation 89), *N*-cyclohexylcarbonyl-β-alanine (Pharmaceutical Preparation 90), and *N-*monomethyl-β-alanine (Pharmaceutical Preparation 91).

### Pharmaceutical Preparation 92; Wrinkle-Improving Jelly

| Component | Amount added (mass%) |
|---|---|
| 95% Ethanol | 10.0 |
| Dipropylene glycol | 15.0 |
| Polyoxyethylene (50 mol) oleyl alcohol ether | 2.0 |
| Carboxyvinyl polymer | 1.0 |
| Sodium hydroxide | 0.15 |
| *L*-arginine | 0.1 |
| N-benzoyl-β-alanine | 1.0 |
| Methylparaben | 0.2 |
| Fragrance | As needed |
| Deionized water | Balance |

### (Production method)

Carboxyvinyl polymer was uniformly dissolved in deionized water, after which dipropylene glycol was added (aqueous phase). Meanwhile, N-benzoyl-β-alanine and polyoxyethylene (50 mol) oleyl alcohol ether were dissolved in 95% ethanol, and the product was added to the aqueous phase. Methylparaben and fragrance were then added, the mixture was neutralized and thickened using sodium hydroxide and L-arginine, and [the product] was manufactured.

### Pharmaceutical Preparations 93 through 109; Wrinkle Improving Jelly

The wrinkle-improving jellies of pharmaceutical preparations 93 through 109 were prepared as in pharmaceutical preparation 92 by mixing the following components added in place of *N*-benzoyl-β-alanine of the components of Pharmaceutical Preparation 92: *N*-(4'-methoxybenzoyl)-β-alanine (Pharmaceutical Preparation 93), *N*-(3'-methoxybenzoyl)-β-alanine (Pharmaceutical Preparation 94), *N*-(2'-methoxybenzoyl)-β-alanine (Pharmaceutical Preparation 95), 3-(1'-piperidine)-propionic acid (Pharmaceutical Preparation 96), *N*-benzyl-β-alanine (Pharmaceutical Preparation 97), *N*-benzenesulfonyl-β-alanine (Pharmaceutical-Preparation 98), *N*-cyclohexyl-β-alanine (Pharmaceutical Preparation 99), *N-*cyclohexylmethyl-β-alanine (Pharmaceutical Preparation 100), *N*-cyclohexyl-*N-*methyl-β-alanine (Pharmaceutical Preparation 101), *N*-(2'-pyridyl)-β-alanine (Pharmaceutical Preparation 102), *N*-nicotinoyl-β-alanine (Pharmaceutical Preparation 103), *N*-benzyloxycarbonyl-β-alanine (Pharmaceutical Preparation 104), β-alanine amide hydrochloride (Pharmaceutical Preparation 105), *N*-3',4',5'-trimethoxybenzoyl-β-alanine (Pharmaceutical Preparation 106), *N*-phenylacetyl-β-alanine (Pharmaceutical Preparation 107), *N*-cyclohexylcarbonyl-β-alanine (Pharmaceutical Preparation 108), and *N*-monomethyl-β-alanine (Pharmaceutical Preparation 109).

### Pharmaceutical Preparation 110; Wrinkle-Improving Clarifying Lotion

| Component | Amount added (mass%) |
|---|---|
| (Phase A) | |
| Ethanol (95%) | 10.0 |
| Polyoxyethylene (20 mol) octyl dodecanol | 1.0 |
| Methylparaben | 0.15 |
| Pantothenyl ethyl ether | 0.1 |
| *N*-benzoyl-β-alanine | 0.05 |
| (Phase B) | |
| Potassium hydroxide | 0.1 |
| (Phase C) | |
| Glycerin | 5.0 |
| Dipropylene glycol | 10.0 |
| Sodium bisulfite | 0.03 |
| Carboxyvinyl polymer | 0.2 |
| Purified water | Balance |

(Production method)
Phases A and C were uniformly dissolved, and phase A was added to phase C to obtain a microemulsion. Then phase B was added, after which packing was performed, and [the product] was manufactured.

### Pharmaceutical Preparations 111 through 127; Wrinkle Improving Clarifying Lotion

The wrinkle-improving clarifying lotions of pharmaceutical preparations 111 through 127 were prepared as in pharmaceutical preparation 110 by mixing the following components added in place of *N*-benzoyl-β-alanine of the components of Pharmaceutical Preparation 110: *N*-(4'-methoxybenzoyl)-β-alanine (Pharmaceutical Preparation 111), *N*-(3'-methoxybenzoyl)-β-alanine (Pharmaceutical Preparation 112), *N*-(2'-methoxybenzoyl)-β-alanine (Pharmaceutical Preparation 113), 3-(1'-piperidine)-propionic acid (Pharmaceutical Preparation 114), *N*-benzyl-β-alanine (Pharmaceutical Preparation 115), *N*-benzenesulfonyl-β-alanine (Pharmaceutical Preparation 116), *N*-cyclohexyl-β-alanine (Pharmaceutical Preparation 117), *N-*cyclohexylmethyl-β-alanine (Pharmaceutical Preparation 118), *N*-cyclohexyl-N-methyl-β-alanine (Pharmaceutical Preparation 119), *N*-(2'-pyridyl)-β-alanine (Pharmaceutical Preparation 120), *N*-nicotinoyl-β-alanine (Pharmaceutical Preparation 121), *N*-benzyloxycarbonyl-β-alanine (Pharmaceutical Preparation 122), β-alanine amide hydrochloride (Pharmaceutical Preparation 123), *N*-3',4',5'-trimethoxybenzoyl-β-alanine (Pharmaceutical Preparation 124), *N*-phenylacetyl-β-alanine (Pharmaceutical Preparation 125), *N*-cyclohexylcarbonyl-β-alanine (Pharmaceutical Preparation 126), and *N*-monomethyl-β-alanine (Pharmaceutical Preparation 127).

### Pharmaceutical Preparation 128; Wrinkle-Improving Pack

| Component | Amount added (mass%) |
|---|---|
| (Phase A) | |
| Dipropylene glycol | 5.0 |
| Polyoxyethylene (60 mol) hydrogenated | 5.0 |
| castor oil | |

| (Phase B) | |
|---|---|
| *N*-benzoyl-β-alanine | 1.0 |
| Olive oil | 5.0 |
| Tocopherol acetate | 0.2 |
| Ethylparaben | 0.2 |
| Fragrance | 0.2 |

| (Phase C) | |
|---|---|
| Sodium bisulfite | 0.03 |
| Polyvinyl alcohol (degree of | 13.0 |
| saponification of 90, degree of | |
| polymerization of 2,000) | |
| Ethanol | 7.0 |
| Purified water | Balance |

(Production Method)
Phases A, B, and C were uniformly dissolved, and phase B was added to phase A to obtain a microemulsion. Next, the microemulsion was added to phase C. Packing was performed, and [the product] was manufactured.

### Pharmaceutical Preparations 129 through 145; Wrinkle Improving Packs

The wrinkle-improving packs of pharmaceutical preparations 129 through 145 were prepared as in pharmaceutical preparation 128 by mixing the following components added in place of *N*-benzoyl-β-alanine of the components of Pharmaceutical Preparation 128: *N*-(4'-methoxybenzoyl)-β-alanine (Pharmaceutical Preparation 129), *N*-(3'-methoxybenzoyl)-β-alanine (Pharmaceutical Preparation 130), *N*-(2'-methoxybenzoyl)-β-alanine (Pharmaceutical Preparation 131), 3-(1'-piperidine)-propionic acid (Pharmaceutical Preparation 132), *N*-benzyl-β-alanine (Pharmaceutical Preparation 133), *N*-benzenesulfonyl-β-alanine (Pharmaceutical Preparation 134), *N*-cyclohexyl-β-alanine (Pharmaceutical Preparation 135), *N-*cyclohexylmethyl-β-alanine (Pharmaceutical Preparation 136), *N*-cyclohexyl-*N-*methyl-β-alanine (Pharmaceutical Preparation 137), *N*-(2'-pyridyl)-β-alanine (Pharmaceutical Preparation 138), *N*-nicotinoyl-β-alanine (Pharmaceutical Preparation 139), *N*-benzyloxycarbonyl-β-alanine (Pharmaceutical Preparation 140), β-alanine amide hydrochloride (Pharmaceutical Preparation 141), *N*-3',4',5'-trimethoxybenzoyl-β-alanine (Pharmaceutical Preparation 142), *N*-phenylacetyl-β-alanine (Pharmaceutical Preparation 143), *N*-cyclohexylcarbonyl-β-alanine (Pharmaceutical Preparation 144), and *N*-monomethyl-β-alanine (Pharmaceutical Preparation 145).

### Pharmaceutical Preparation 146; Wrinkle-Improving Ointment

| Component | Amount added (mass%) |
|---|---|
| Polyoxyethylene (30 mol) cetyl ether | 2.0 |
| Glycerin monostearate | 10.0 |
| Liquid paraffin | 10.0 |
| Vaseline | 40.0 |
| Cetanol | 6.0 |
| Methylparaben | 0.1 |
| Butylparaben | 0.1 |
| Glycerin monostearic acid ester | 2.0 |
| *N*-benzoyl-β-alanine | 5.0 |
| Propylene glycol | 10.0 |
| Deionized water | Balance |
| Fragrance | As needed |

### (Production Method)

Propylene glycol was added to deionized water and dissolved, heated, and maintained at 70°C (aqueous phase). The other components were mixed and dissolved at 70°C (oil phase). The oil phase was added to the aqueous phase, uniformly emulsified using a homomixer, and cooled to 30°C. Packing was then performed, and [the product] was manufactured.

### Pharmaceutical Preparations 147 through 163; Wrinkle Improving Ointments

The wrinkle-improving ointments of pharmaceutical preparations 147 through 163 were prepared as in pharmaceutical preparation 146 by mixing the following components added in place of *N*-benzoyl-β-alanine of the components of Pharmaceutical Preparation 146: *N*-(4'-methoxybenzoyl)-β-alanine (Pharmaceutical Preparation 147), *N*-(3'-methoxybenzoyl)-β-alanine (Pharmaceutical Preparation 148), *N*-(2'-methoxybenzoyl)-β-alanine (Pharmaceutical Preparation 149), 3-(1'-piperidine)-propionic acid (Pharmaceutical Preparation 150), *N*-benzyl-β-alanine (Pharmaceutical Preparation 151), *N*-benzenesulfonyl-β-alanine (Pharmaceutical Preparation 152), *N*-cyclohexyl-β-alanine (Pharmaceutical Preparation 153), *N-*cyclohexylmethyl-β-alanine (Pharmaceutical Preparation 154), *N*-cyclohexyl-*N-*methyl-β-alanine (Pharmaceutical Preparation 155), *N*-(2'-pyridyl)-β-alanine (Pharmaceutical Preparation 156), *N*-nicotinoyl-β-alanine (Pharmaceutical Preparation 157), *N*-benzyloxycarbonyl-β-alanine (Pharmaceutical Preparation 158), β-alanine amide hydrochloride (Pharmaceutical Preparation 159), *N*-3',4',5'-trimethoxybenzoyl-β-alanine (Pharmaceutical Preparation 160), *N*-phenylacetyl-β-alanine (Pharmaceutical Preparation 161), *N*-cyclohexylcarbonyl-β-alanine (Pharmaceutical Preparation 162), and *N*-monomethyl-β-alanine (Pharmaceutical Preparation 163).

### Pharmaceutical Preparation 164; Wrinkle Improving Cream

| Component | Amount added (mass%) |
|---|---|
| (1) Liquid paraffin | 8.0 |
| (2) Vaseline | 3.0 |
| (3) Dimethylpolysiloxane | 2.0 |
| (4) Stearyl alcohol | 3.0 |
| (5) Behenyl alcohol | 2.0 |
| (6) Glycerin | 5.0 |
| (7) Dipropylene glycol | 4.0 |
| (8) Trehalose | 1.0 |
| (9) Pentaerythritol tetra-2-ethylhexanoate | 4.0 |
| (10) Polyoxyethylene glyceryl monoisostearate | 2.0 |
| (11) Polyoxyethylene glycerin monostearate | 1.0 |
| (12) Lipophilic glycerin monostearate | 2.0 |
| (13) Citric acid | 0.05 |
| (14) Sodium citrate | 0.05 |
| (15) Potassium hydroxide | 0.015 |
| (16) Oil-soluble licorice extract | 0.1 |
| (17) Retinol palmitate (1 million units) | 0.25 |
| (18) *N*-benzoyl-β-alanine | 1.0 |
| (19) Tocopherol acetate | 0.1 |
| (20) Parahydroxybenzoic acid ester | As needed |
| (21) Phenoxyethanol | As needed |
| (22) Dibutylhydroxytoluene | As needed |
| (23) Edetate trisodium | 0.05 |
| (24) 4-t-butyl-4'-methoxybenzoylmethane | 0.01 |
| (25) 2-ethylhexyl paramethoxycinnamate | 0.1 |
| (26) β-carotene | 0.01 |
| (27) Polyvinyl alcohol | 0.5 |
| (28) Hydroxyethylcellulose | 0.5 |
| (29) Carboxyvinyl polymer | 0.05 |
| (30) Purified water | Balance |
| (31) Fragrance | As needed |

### (Production Method)

(27) through (29) were dissolved in purified water (30). (6) through (8), (13), (14), (18), (21), and (23) through (25) were then added, and the resulting mixture was heated and kept at 70°C (aqueous phase). (1) through (5), (9) through (12), (16), (17), (19), (20), (22), (26), and (31) were mixed and dissolved (oil phase). The oil phase was added to the aqueous phase and uniformly mixed. (15) was then added, and the mixture was neutralized. Cooling and packing were then performed, and [the product] was manufactured.

### Pharmaceutical Preparations 165 through 181; Wrinkle Improving Creams

The wrinkle-improving creams of pharmaceutical preparations 165 through 181 were prepared as in pharmaceutical preparation 164 by mixing the following components added in place of *N*-benzoyl-β-alanine of the components of Pharmaceutical Preparation 164: *N*-(4'-methoxybenzoyl)-β-alanine (Pharmaceutical Preparation 165), *N*-(3'-methoxybenzoyl)-β-alanine (Pharmaceutical Preparation 166), *N*-(2'-methoxybenzoyl)-β-alanine (Pharmaceutical Preparation 167), 3-(1'-piperidine)-propionic acid (Pharmaceutical Preparation 168), *N*-benzyl-β-alanine (Pharmaceutical Preparation 169), *N*-benzenesulfonyl-β-alanine (Pharmaceutical Preparation 170), *N*-cyclohexyl-β-alanine (Pharmaceutical Preparation 171), N-cyclohexylmethyl-β-alanine (Pharmaceutical Preparation 172), *N*-cyclohexyl-*N-*methyl-β-alanine (Pharmaceutical Preparation 173), *N*-(2'-pyridyl)-β-alanine (Pharmaceutical Preparation 174), *N*-nicotinoyl-β-alanine (Pharmaceutical Preparation 175), *N*-benzyloxycarbonyl-β-alanine (Pharmaceutical Preparation 176), β-alanine amide hydrochloride (Pharmaceutical Preparation 177), *N*-3',4',5'-trimethoxybenzoyl-β-alanine (Pharmaceutical Preparation 178), *N*-phenylacetyl-β-alanine (Pharmaceutical Preparation 179), *N*-cyclohexylcarbonyl-β-alanine (Pharmaceutical Preparation 180), and *N*-monomethyl-β-alanine (Pharmaceutical Preparation 181).

### Pharmaceutical Preparation 182; Wrinkle Improving Cream

| Component | Amount added (mass%) |
|---|---|
| (1) Vaseline | 2.0 |
| (2) Dimethylpolysiloxane | 2.0 |
| (3) Ethanol | 5.0 |
| (4) Behenyl alcohol | 0.5 |
| (5) Batyl alcohol | 0.2 |
| (6) Glycerin | 7.0 |
| (7) 1,3-butylene glycol | 5.0 |
| (8) Polyethylene glycol 20,000 | 0.5 |
| (9) Jojoba oil | 3.0 |
| (10) Squalane | 2.0 |
| (11) Phytosteryl hydroxystearate | 0.5 |
| (12) Pentaerythritol tetra-2-ethylhexanoate | 1.0 |
| (13) Polyoxyethylene hydrogenated castor oil | 1.0 |
| (14) Potassium hydroxide | 0.1 |
| (15) Sodium pyrosulfite | 0.01 |
| (16) Sodium hexametaphosphate | 0.05 |
| (17) Stearyl glyceryl retinoic acid | 0.1 |
| (18) Pantothenylethyl ether | 0.1 |
| (19) Arbutin | 7.0 |
| (20) Methylamide hydrochloride tranexamate | 1.0 |
| (21) *N*-benzoyl-β-alanine | 1.0 |
| (22) Tocopherol acetate | 0.1 |
| (23) Sodium hyaluronate | 0.05 |
| (24) Parahydroxybenzoic acid ester | As needed |
| (25) Trisodium edetate | 0.05 |
| (26) 4-t-butyl-4'-methoxybenzoylmethane | 0.1 |
| (27) Mono-2-ethylhexanoic acid glyceryl diparamethoxycinnamate | 0.1 |
| (28) Yellow iron oxide | As needed |
| (29) Xanthan gum | 0.1 |
| (30) Carboxyvinyl polymer | 0.2 |
| (31) Purified water | Balance |

### (Production Method)

(29) and (30) were dissolved in purified water (31). (3), (6) through (8), (15), (16), (18) through (21), and (23) were then added, and the mixture was heated and maintained at 70°C (aqueous phase). (1), (2), (4), (5), (9) through (13), (17), (22), (24), (26), and (27) were mixed and dissolved (oil phase). (25) and (28) were added to a small amount of purified water (31) and dispersed using a homomixer (pigment). The oil phase and the pigment were added to the aqueous phase and uniformly mixed. (14) was then added, and the mixture was neutralized. Cooling and packing were then performed, and [the product] was manufactured.

### Pharmaceutical Preparations 183 through 199; Wrinkle Improving Creams

The wrinkle-improving creams of pharmaceutical preparations 183 through 199 were prepared as in pharmaceutical preparation 182 by mixing the following components added in place of *N*-benzoyl-β-alanine of the components of Pharmaceutical Preparation 182: *N*-(4'-methoxybenzoyl)-β-alanine (Pharmaceutical Preparation 183), *N*-(3'-methoxybenzoyl)-β-alanine (Pharmaceutical Preparation 184), *N*-(2'-methoxybenzoyl)-β-alanine (Pharmaceutical Preparation 185), 3-(1'-piperidine)-propionic acid (Pharmaceutical Preparation 186), *N*-benzyl-β-alanine (Pharmaceutical Preparation 187), *N*-benzenesulfonyl-β-alanine (Pharmaceutical Preparation 188), *N*-cyclohexyl-β-alanine (Pharmaceutical Preparation 189), *N-*cyclohexylmethyl-β-alanine (Pharmaceutical Preparation 190), *N*-cyclohexyl-*N-*methyl-β-alanine (Pharmaceutical Preparation 191), *N*-(2'-pyridyl)-β-alanine (Pharmaceutical Preparation 192), *N*-nicotinoyl-β-alanine (Pharmaceutical Preparation 193), *N*-benzyloxycarbonyl-β-alanine (Pharmaceutical Preparation 194), β-alanine amide hydrochloride (Pharmaceutical Preparation 195), *N*-3',4',5'-trimethoxybenzoyl-β-alanine (Pharmaceutical Preparation 196), *N*-phenylacetyl-β-alanine (Pharmaceutical Preparation 197), *N*-cyclohexylcarbonyl-β-alanine (Pharmaceutical Preparation 198), and *N*-monomethyl-β-alanine (Pharmaceutical Preparation 199).

### INDUSTRIAL APPLICABILITY

The β-alanine derivatives represented by general formula (1), (2), or (3) according to the present invention, and salts thereof have the excellent function of improving wrinkles; therefore, these compounds are used in pharmaceutical products, food products, and cosmetics containing quasidrugs; and various other fields as wrinkle-improving agents.

## Claims

1. A wrinkle-improving agent comprising as an active component one, two, or more compounds selected from β-alanine derivatives represented by the following general formula (1), (2) or (3), and salts thereof. 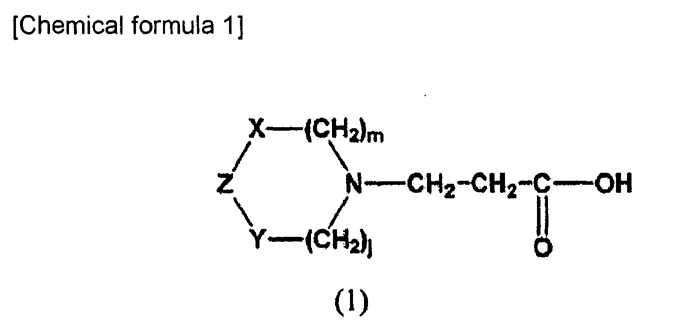 (In general formula (1), X and Y each independently represent an oxygen atom (O), a nitrogen atom (N), a CHQ group (in the group, Q represents a hydrogen atom (H) or a C₁₋₃ alkyl group or hydroxyl group), or a carbonyl group (C=O). Z represents a methylene group (CH₂), an ethylene group (CH₂-CH₂), CH=CH, or a benzene ring. j and m each independently represent an integer of 0 to 5. The total of j and m (j + m) is 0 to 6.) 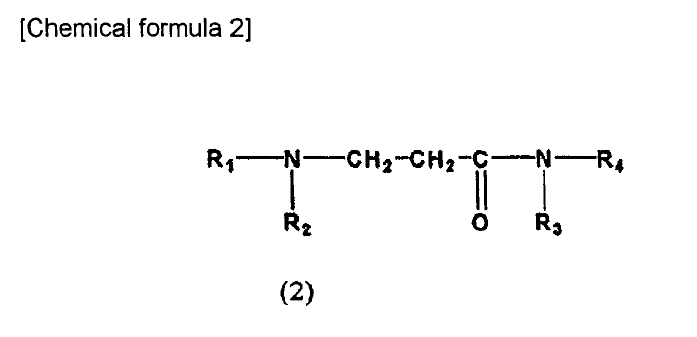 (In general formula (2), R₁ and R₂ each independently represent a hydrogen atom, C₁₋₃ alkyl group, or C₁₋₃ alkenyl group. R₃ and R₄ each independently represent a hydrogen atom, a C₁₋₃ alkyl group, a C₁₋₃ alkenyl group, a cyclohexyl group, or a group containing nitrogen atoms and forming a cyclic structure by R₃ and R₄. The total number of carbons in R₃ and R₄ is 0 to 6.) 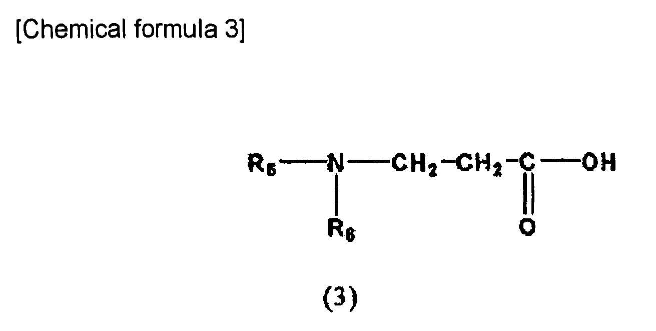 [In general formula (3), R₅ and R₆ are each independently a hydrogen atom, C₁₋₇ alkyl group, C₁₋₇ alkenyl group, C₃₋₇ hydrocarbon group having a cyclic segment, pyridyl group, cyclohexyl carbonyl group, cyclopentyl carbonyl group, nicotinoyl group, isonicotinoyl group, picolinoyl group, nipecotinoyl group, isonipecotinoyl group, N-acetyl nipecotinoyl group, N-acetyl isonipecotinoyl group, benzyloxycarbonyl group, group represented by general formula (4) 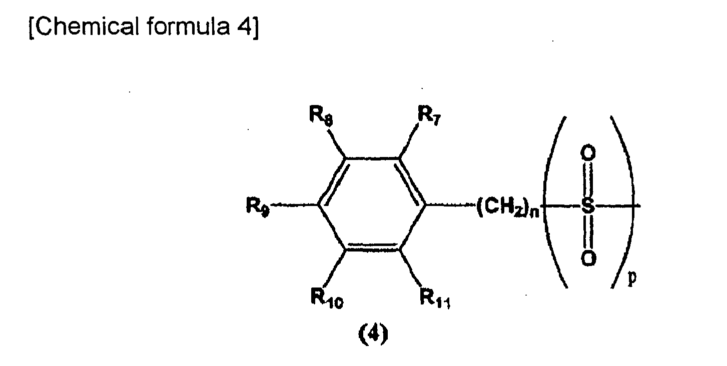 (In general formula (4), R₇ through R₁₁ each independently represent a hydrogen atom, hydroxyl group, C₁₋₄ alkyloxy group, C₁₋₄ alkenyloxy group, C₁₋₃ alkyl group, or C₁₋₃ alkenyl group; n represents an integer of 0 to 2; and p represents an integer of 0 or 1), or a group represented by general formula (5) 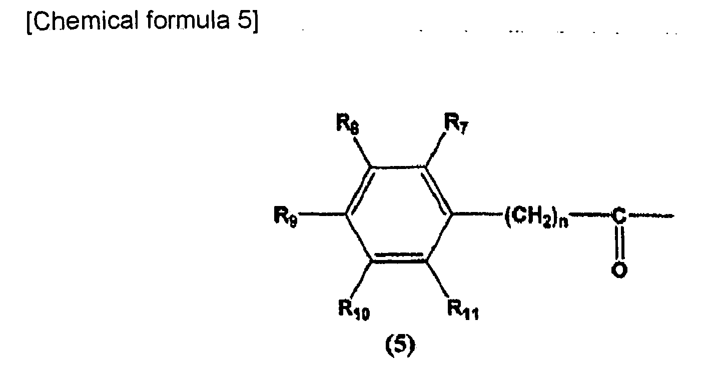 (in general formula (5), R₇ through R₁₁ and n are the same as in general formula (4)). R₅ and R₆ cannot both be hydrogen atoms.]

2. The wrinkle-improving agent of claim 1, where the β-alanine derivative of claim 1 is N-benzoyl-β-alanine.
